# EUROPEAN PATENT APPLICATION

(11) **EP 1 277 730 A1**
(43) Date of publication of application: **22.01.2003**
(21) Application number: 01917499.4
(22) Date of filing: 27.03.2001
(51) Int. Cl.: C07C 251/54, C07D 263/32, A61K 31/195, A61K 31/421, A61P 29/00, A61P 35/00, A61P 9/10, A61P 27/14

(54) **NEOVASCULARIZATION INHIBITORS**

(30) Priority: 28.03.2000 JP 2000092966
(71) Applicant: Takeda Chemical Industries, Ltd., Osaka-shi, Osaka 541-0045 (JP)
(72) Inventor: HAZAMA, Masatoshi, Ikeda-shi, Osaka 563-0029 (JP); MIYAZAKI, Takeshi, Toyono-gun, Osaka 563-0103 (JP); SUGIYAMA, Yasuo, Kawanishi-shi, Hyogo 666-0111 (JP)
(74) Representative: Wright, Robert Gordon McRae
(86) International application number: JP0102447
(87) International publication number: WO01072695

(57) **Abstract**

An angiogenesis inhibitor containing a compound represented by the formula 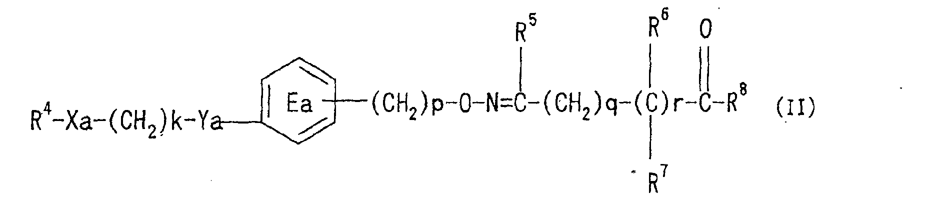 wherein R⁴ is an optionally substituted hydrocarbon group and the like; Xa is a bond and the like; k is an integer of 1 to 3; Ya is an oxygen atom and the like; ring Ea is a benzene ring optionally having additional substituent(s); p is an integer of 1 to 8; R⁵ is a hydrogen atom and the like; q is an integer of 0 to 6; r is 0 or 1; R⁸ is a hydroxy group and the like; and R⁶ and R⁷ are hydrogen atoms and the like, or a salt thereof is useful as an agent for the prophylaxis or treatment of tumor and the like.

## Description

### Technical Field

The present invention relates to an angiogenesis inhibitor useful as an agent for the prophylaxis or treatment of tumor and the like.

### Background Art

There are many diseases wherein angiogenesis, a phenomenon to newly construct a vascular system, is involved in the onset and progress thereof, and solid tumor, diabetic retinopathy and inflammatory disease (rheumatism etc.) are the representative examples. For a solid tumor to grow, the route for supply of nutrition and oxygen, and the route for removal of waste products need to be ensured by angiogenesis. In metastasis, which is a major problem in the current treatments of cancer, angiogenesis is an important way of ensuring such route. In diabetic retinopathy, angiogenesis itself is a disease state, and when let untreated, it leads to a blindness. Therefore, suppression of angiogenesis is considered to afford prophylaxis or treatment of the above-mentioned diseases and an angiogenesis inhibitor has been sought for. For this end, investigation of the clinical effectiveness of some substances is ongoing [Folkman. J., Scientific American, p. 116, September (1996)].

Thus, there is a demand for the development of an angiogenesis inhibitor having a superior action and low toxicity.

### Disclosure of the Invention

The present invention relates to
(1) an angiogenesis inhibitor containing a compound represented by the formula 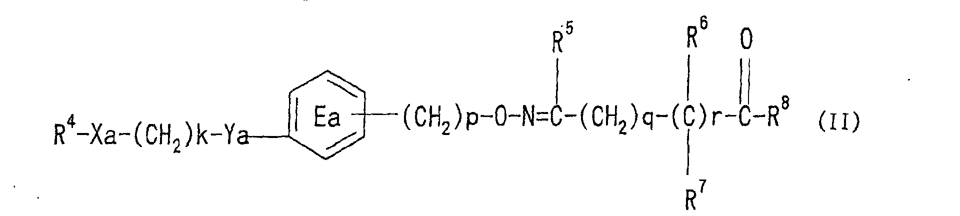 wherein
   - R⁴: is an optionally substituted hydrocarbon group or an optionally substituted heterocyclic group;
   - Xa: is a bond, a group represented by the formula: -CO-, -CH(OH)- or -NR⁹- (R⁹ is a hydrogen atom or an optionally substituted alkyl group);
   - k: is an integer of 1 to 3;
   - Ya: is an oxygen atom, a sulfur atom, a group represented by the formula: -SO-, -SO₂- or -NR¹⁰- (R¹⁰ is a hydrogen atom or an optionally substituted alkyl group);
   - ring Ea: is a benzene ring optionally having additional substituent(s);
   - p: is an integer of 1 to 8;
   - R⁵: is a hydrogen atom, an optionally substituted hydrocarbon group or an optionally substituted heterocyclic group;
   - q: is an integer of 0 to 6;
   - r: is 0 or 1;
   - R⁸: is a hydroxy group, -OR¹¹ (R¹¹ is an optionally substituted hydrocarbon group) or -NR¹²R¹³ (R¹² and R¹³ are the same or different and each is a hydrogen atom, an optionally substituted hydrocarbon group, an optionally substituted heterocyclic group, or an acyl group, or R¹² and R¹³ are optionally bonded to form a ring); and
   - R⁶ and R⁷: are the same or different and each is a hydrogen atom, an optionally substituted hydrocarbon group, or R⁶ and R⁵ are optionally bonded to form a ring,
   or a salt thereof (hereinafter sometimes to be referred to as compound (II));
(2) the inhibitor of the above-mentioned (1), which contains E-4-[4-(5-methyl-2-phenyl-4-oxazolylmethoxy)benzyloxyimino]-4-phenylbutyric acid or a salt thereof;
(3) a method for inhibiting angiogenesis in a mammal, which comprises administering compound (II) to the mammal;
(4) use of compound (II) for the production of an angiogenesis inhibitor; and the like.

In the formula (II), as the "optionally substituted hydrocarbon group or an optionally substituted heterocyclic group" represented by R⁴, for example, those exemplified for R in the formula (I) to be mentioned below are used.

R⁴ is preferably an optionally substituted heterocyclic group, more preferably an optionally substituted pyridyl, an optionally substituted oxazolyl, an optionally substituted thiazolyl or an optionally substituted triazolyl. R⁴ is particularly preferably pyridyl, oxazolyl, thiazolyl or triazolyl, each of which optionally has 1 or 2 substituents selected from the group consisting of an alkyl having 1 to 3 carbon atoms, a cycloalkyl having 3 to 7 carbon atoms, a furyl, a thienyl, a phenyl and a naphthyl. As used herein, the furyl, thienyl, phenyl and naphthyl optionally have 1 or 2 substituents selected from an alkyl having 1 to 3 carbon atoms, an alkoxy having 1 to 3 carbon atoms, a halogen (e.g., fluorine, chlorine, bromine, iodine etc.) and a haloalkyl having 1 to 3 carbon atoms.

In the formula (II), as the "optionally substituted alkyl group" represented by R⁹ for Xa, those exemplified as R³ for Y in the formula (I) to be mentioned below are used.

In the formula (II), k is an integer of 1 to 3, preferably 1 or 2.

In the formula (II), Ya is -O-, -S-, -SO-, -SO₂- or -NR¹⁰-(R¹⁰ is a hydrogen atom or an optionally substituted alkyl group), preferebaly -O-, -S- or -NR¹⁰- wherein, as the "optionally substituted alkyl group" for R¹⁰, those exemplified as R³ for Y in the formula (I) are used.

In the formula (II), as the substituent for the "benzene ring optionally having additional substituent(s)" represented by ring Ea, those exemplified as the substituents for the ring E in the formula (I) to be mentioned below are used. The substituents preferably include an alkyl group having 1 to 4 carbon atoms, an alkoxyl group having 1 to 4 carbon atoms and a halogen atom. The number of substituents is preferably 1 to 3.

In the formula (II), p is preferably an integer of 1 to 3.

In the formula (II), as the "optionally substituted hydrocarbon group or an optionally substituted heterocyclic group" represented by R⁵, for example, one exemplified for R in the formula (I) to be mentioned below are used.

R⁵ is preferably an optionally substituted hydrocarbon group. R⁵ is more preferably an optionally substituted alkyl group having 1 to 4 carbon atoms, an optionally substituted phenylalkenyl having 8 to 10 carbon atoms or an optionally substituted aryl group having 6 to 14 carbon atoms. The substituents these hydrocarbon groups may have are preferably a halogen atom, an alkoxyl group having 1 to 4 carbon atoms, an aryloxy group having 6 to 14 carbon atoms and an aromatic heterocyclic group (e.g., furyl, thienyl).

In the formula (II), q is preferably an integer of 0 to 4.

As regards R⁸ in the formula (II), as the "optionally substituted hydrocarbon group" represented by R¹¹, for example, those exemplified for R in the formula (I) to be mentioned below are used.

R¹¹ is preferably an "alkyl group having 1 to 4 carbon atoms" or an "aryl group having 6 to 10 carbon atoms (preferably pheny) which is optionally substituted by an alkyl group having 1 to 4 carbon atoms (preferably methyl or ethyl) or a halogen atom (preferably chlorine)".

As regards R⁸ in the formula (II), as the "optionally substituted hydrocarbon group" and the "optionally substituted heterocyclic group" represented by R¹² and R¹³, for example, those exemplified for R in the formula (I) to be mentioned below are used.

As the "acyl group" represented by R¹² and R¹³, for example, those exemplified for R in the formula (I) to be mentioned below are used.

The ring to be formed by R¹² and R¹³ in combination is, for example, a 5 to 7-membered cyclic amino group. Particularly, 1-pyrrolidinyl, 1-piperidinyl, 1-hexamethyleniminyl, 4-morpholino, 4-thiomorpholino and the like are preferable.

As the "optionally substituted hydrocarbon group" represented by R⁶ and R⁷ in the formula (II), for example, those exemplified for R in the formula (I) to be mentioned below are used. Particularly, those similar to the "optionally substituted alkyl group" exemplified as R³ for Y in the formula (I) to be mentioned below are preferable.

Examples of the ring formed by R⁶ and R⁵ bonded to each other include a cycloalkane having 5 to 11 carbon atoms, a cycloalkene having 5 to 11 carbon atoms and the like, and cyclopentane, cyclopentene, cyclohexane, cyclohexene, cycloheptane, cycloheptene, cyclooctane, cyclooctene, cyclononane, cyclononene, cyclodecane, cyclodecene, cycloundecane, cycloundecene and the like are specifically mentioned.

The compound represented by the formula (II) includes an (E) form and a (Z) form around an imino bond. This compound includes these (E) form and (Z) form as single compounds, or a mixture thereof.

Preferable examples of the compounds represented by the formula (II) are the following compounds (1)-(10) and the like.
(1) Z-2-[4-(5-methyl-2-phenyl-4-oxazolylmethoxy)benzyloxy-imino]-2-phenylacetic acid;
(2) Z-4-[4-(5-methyl-2-phenyl-4-oxazolylmethoxy)benzyloxy-imino]-4-phenylbutyric acid;
(3) Z-2-(4-bromophenyl)-2-[4-(5-methyl-2-phenyl-4-oxazolylmethoxy)benzyloxyimino]acetic acid;
(4) Z-2-[4-(5-methyl-2-phenyl-4-oxazolylmethoxy)benzyloxy-imino]-2-(4-phenoxyphenyl)acetic acid;
(5) Z-4-(4-fluorophenyl)-4-[4-(5-methyl-2-phenyl-4-oxazolylmethoxy)benzyloxyimino]butyric acid;
(6) Z-3-methyl-2-[4-(5-methyl-2-phenyl-4-oxazolylmethoxy)-benzyloxyimino]butyric acid;
(7) E-4-[4-(5-methyl-2-phenyl-4-oxazolylmethoxy)benzyloxy-imino]-4-phenylbutyric acid;
(8) E-4-(4-fluorophenyl)-4-[4-(5-methyl-2-phenyl-4-oxazolylmethoxy)benzyloxyimino]butyric acid;
(9) E-4-[4-(5-methyl-2-phenyl-4-oxazolylmethoxy)-benzyloxyimino]-4-phenylbutylamide;
(10) E-8-[4-(5-methyl-2-phenyl-4-oxazolylmethoxy)benzyloxy-imino]-8-phenyloctanoic acid.

These compounds are hereafter sometimes to be simply referred to as compound (1), compound (2) and so on.

As the salt of the compound represented by the formula (II), those similar to the salts of the compound represented by the formula (I) to be mentioned below are used. Particularly, sodium salt, potassium salt, hydrochloride and the like are preferable.

The compound represented by the formula (II) and a salt thereof can be produced by, for example, the method described in WO99/58510 or a method analogous thereto.

For the angiogenesis inhibitor of the present invention, compound (II) and an "insulin sensitizer other than compound (II)" may be used in combination, with the aim of "potentiation of the angiogenesis inhibitory action of compound (II)", "reduction of the amount of compound (II) to be used", "reduction of side effects of compound (II)" and the like. The "insulin sensitizer other than compound (II)" is a pharmaceutical agent that improves insulin sensitivity by restoring disordered insulin receptor functions, and is exemplified by a compound represented by the formula 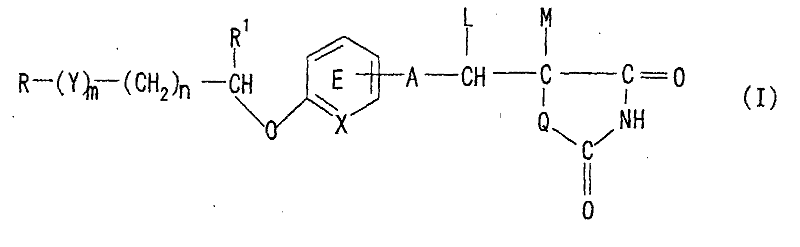 wherein R is an optionally substituted hydrocarbon group or an optionally substituted heterocyclic group; Y is a group represented by the formula -CO-, -CH(OH)- or -NR³- (R³ is an optionally substituted alkyl group); m is 0 or 1; n is 0, 1 or 2; X is CH or N; A is a bond or a divalent aliphatic hydrocarbon group; Q is an oxygen atom or a sulfur atom; and R¹ is a hydrogen atom or an alkyl group. The ring E may have an additional substituent and the substituent may be bonded to R¹ to form a ring. L and M are each a hydrogen atom or bonded to each other to form a bond, or a salt thereof.

In the formula (I), examples of the hydrocarbon group of the optionally substituted hydrocarbon group represented by R include an aliphatic hydrocarbon group, an alicyclic hydrocarbon group, an alicyclic-aliphatic hydrocarbon group, an aromatic-aliphatic hydrocarbon group and an aromatic hydrocarbon group. These hydrocarbon groups preferably have 1 to 14 carbon atoms.

The aliphatic hydrocarbon group is preferably that having 1 to 8 carbon atoms. Examples of the aliphatic hydrocarbon group include saturated aliphatic hydrocarbon group having 1 to 8 carbon atoms (e.g., alkyl group etc.), such as methyl, ethyl, propyl, isopropyl, butyl, isobutyl, sec.-butyl, t.-butyl, pentyl, isopentyl, neopentyl, t.-pentyl, hexyl, isohexyl, heptyl, octyl and the like; and unsaturated aliphatic hydrocarbon group having 2 to 8 carbon atoms (e.g., alkenyl group, alkadienyl group, alkynyl group, alkadiinyl group etc.), such as vinyl, 1-propenyl, 2-propenyl, 1-butenyl, 2-butenyl, 3-butenyl, 2-methyl-1-propenyl, 1-pentenyl, 2-pentenyl, 3-pentenyl, 4-pentenyl, 3-methyl-2-butenyl, 1-hexenyl, 3-hexenyl, 2,4-hexadienyl, 5-hexenyl, 1-heptenyl, 1-octenyl, ethynyl, 1-propinyl, 2-propinyl, 1-butynyl, 2-butynyl, 3-butynyl, 1-pentinyl, 2-pentinyl, 3-pentinyl, 4-pentinyl, 1-hexynyl, 3-hexynyl, 2,4-hexadiinyl, 5-hexynyl, 1-heptinyl, 1-octynyl and the like.

The alicyclic hydrocarbon group is preferably that having 3 to 7 carbon atoms. Examples of the alicyclic hydrocarbon group include a saturated alicyclic hydrocarbon group having 3 to 7 carbon atoms (e.g., cycloalkyl group etc.), such as cyclopropyl, cyclobutyl, cyclopentyl, cyclohexyl, cycloheptyl and the like; an unsaturated alicyclic hydrocarbon group having 5 to 7 carbon atoms (e.g., cycloalkenyl group, cycloalkadienyl group etc.), such as 1-cyclopentenyl, 2-cyclopentenyl, 3-cyclopentenyl, 1-cyclohexenyl, 2-cyclohexenyl, 3-cyclohexenyl, 1-cycloheptenyl, 2-cycloheptenyl, 3-cycloheptenyl, 2,4-cycloheptadienyl and the like.

Examples of the alicyclic-aliphatic hydrocarbon group include one wherein the above-mentioned alicyclic hydrocarbon group and the aliphatic hydrocarbon group are bonded (e.g., cycloalkyl-alkyl group, cycloalkenyl-alkyl group etc.). Of these, an alicyclic-aliphatic hydrocarbon group having 4 to 9 carbon atoms is preferable. Examples of the alicyclic-aliphatic hydrocarbon group include cyclopropylmethyl, cyclopropylethyl, cyclobutylmethyl, cyclopentylmethyl, 2-cyclopentenylmethyl, 3-cyclopentenylmethyl, cyclohexylmethyl, 2-cyclohexenylmethyl, 3-cyclohexenylmethyl, cyclohexylethyl, cyclohexylpropyl, cycloheptylmethyl, cycloheptylethyl and the like.

The aromatic-aliphatic hydrocarbon group is preferably aromatic-aliphatic hydrocarbon group having 7 to 13 carbon atoms (e.g., aralkyl group etc.). Examples of the aromatic-aliphatic hydrocarbon group include a phenylalkyl having 7 to 9 carbon atoms such as benzyl, phenethyl, 1-phenylethyl, 3-phenylpropyl, 2-phenylpropyl, 1-phenylpropyl and the like; naphthylalkyl having 11 to 13 carbon atoms such as α-naphthylmethyl, α-naphthylethyl, β-naphthylmethyl, β-naphthylethyl and the like.

The aromatic hydrocarbon group is preferably an aromatic hydrocarbon group having 6 to 14 carbon atoms (e.g., aryl group etc.). Examples of the aromatic hydrocarbon group include phenyl, naphthyl (α-naphthyl, β-naphthyl) and the like.

In the formula (I), examples of the heterocyclic group of the optionally substituted heterocyclic group represented by R include a 5 to 7-membered heterocyclic group containing as a ring-constituting atom, besides a carbon atom, 1 to 4 heteroatoms selected from an oxygen atom, a sulfur atom and a nitrogen atom, or a fused ring group. Examples of the fused ring include a fused ring of such 5 to 7-membered heterocycle with a 6-membered ring containing 1 or 2 nitrogen atoms, a benzene ring or a 5-membered ring containing one sulfur atom.

Examples of the heterocyclic group include 2-pyridyl, 3-pyridyl, 4-pyridyl, 2-pyrimidinyl, 4-pyrimidinyl, 5-pyrimidinyl, 6-pyrimidinyl, 3-pyridazinyl, 4-pyridazinyl, 2-pyrazinyl, 2-pyrrolyl, 3-pyrrolyl, 2-imidazolyl, 4-imidazolyl, 5-imidazolyl, 3-pyrazolyl, 4-pyrazolyl, isothiazolyl, isoxazolyl, 2-thiazolyl, 4-thiazolyl, 5-thiazolyl, 2-oxazolyl, 4-oxazolyl, 5-oxazolyl, 1,2,4-oxadiazol-5-yl, 1,2,4-triazol-3-yl, 1,2,3-triazol-4-yl, tetrazol-5-yl, benzimidazol-2-yl, indol-3-yl, 1H-indazol-3-yl 1H-pyrrolo[2,3-b]pyrazin-2-yl, 1H-pyrrolo[2,3-b]pyridin-6-yl, 1H-imidazo[4,5-b]pyridin-2-yl, 1H-imidazo[4,5-c]pyridin-2-yl, 1H-imidazo[4,5-b]pyrazin-2-yl, benzopyranyl, dihydrobenzopyranyl and the like. The heterocyclic group is preferably pyridyl, oxazolyl or thiazolyl group.

In the formula (I), the hydrocarbon group and heterocyclic group represented by R each optionally have 1 to 5, preferably 1 to 3, substituents at optional substitutable positions. Examples of the substituent include an aliphatic hydrocarbon group, an alicyclic hydrocarbon group, an aryl group, an aromatic heterocyclic group, a non-aromatic heterocyclic group, a halogen atom, a nitro group, an optionally substituted amino group, an acyl group, an optionally substituted hydroxyl group, an optionally substituted thiol group, an optionally esterified carboxyl group, an amidino group, a carbamoyl group, a sulfamoyl group, a sulfo group, a cyano group, an azide group and a nitroso group.

Examples of the aliphatic hydrocarbon group include a straight-chain or branched aliphatic hydrocarbon group having 1 to 15 carbon atoms, such as alkyl group, alkenyl group, alkynyl group and the like.

Preferable examples of the alkyl group include an alkyl group having 1 to 10 carbon atoms, such as methyl, ethyl, propyl, isopropyl, butyl, isobutyl, sec.-butyl, t.-butyl, pentyl, isopentyl, neopentyl, t.-pentyl, 1-ethylpropyl, hexyl, isohexyl, 1,1-dimethylbutyl, 2,2-dimethylbutyl, 3,3-dimethylbutyl, 2-ethylbutyl, hexyl, pentyl, octyl, nonyl, decyl and the like.

Preferable examples of the alkenyl group include an alkenyl group having 2 to 10 carbon atoms, such as vinyl, allyl, isopropenyl, 1-propenyl, 2-methyl-1-propenyl, 1-butenyl, 2-butenyl, 3-butenyl, 2-ethyl-1-butenyl, 3-methyl-2-butenyl, 1-pentenyl, 2-pentenyl, 3-pentenyl, 4-pentenyl, 4-methyl-3-pentenyl, 1-hexenyl, 2-hexenyl, 3-hexenyl, 4-hexenyl, 5-hexenyl and the like.

Preferable examples of the alkynyl group include an alkynyl group having 2 to 10 carbon atoms, such as ethynyl, 1-propinyl, 2-propinyl, 1-butynyl, 2-butynyl, 3-butynyl, 1-pentinyl, 2-pentinyl, 3-pentinyl, 4-pentinyl, 1-hexynyl, 2-hexynyl, 3-hexynyl, 4-hexynyl, 5-hexynyl and the like.

Examples of the alicyclic hydrocarbon group include a saturated or unsaturated alicyclic hydrocarbon group having 3 to 12 carbon atoms, such as cycloalkyl group, cycloalkenyl group, cycloalkadienyl group and the like.

Preferable examples of the cycloalkyl group include a cycloalkyl group having 3 to 10 carbon atoms, such as cyclopropyl, cyclobutyl, cyclopentyl, cyclohexyl, cycloheptyl, cyclooctyl, bicyclo[2.2.1]heptyl, bicyclo[2.2.2]octyl, bicyclo[3.2.1]octyl, bicyclo[3.2.2]nonyl, bicyclo[3.3.1]nonyl, bicyclo[4.2.1]nonyl, bicyclo[4.3.1]decyl and the like.

Preferable examples of the cycloalkenyl group include cycloalkenyl group having 3 to 10 carbon atoms, such as 2-cyclopenten-1-yl, 3-cyclopenten-1-yl, 2-cyclohexen-1-yl, 3-cyclohexen-1-yl and the like.

Preferable examples of the cycloalkadienyl group include a cycloalkadienyl group having 4 to 10 carbon atoms, such as 2,4-cyclopentadien-1-yl, 2,4-cyclohexadien-1-yl, 2,5-cyclohexadien-1-yl and the like.

Preferable examples of the aryl group include an aryl group having 6 to 14 carbon atoms, such as phenyl, naphthyl (1-naphthyl and 2-naphthyl), anthryl, phenanthryl, acenaphtylenyl and the like.

Preferable examples of the aromatic heterocyclic group include an aromatic monocyclic heterocyclic group, such as furyl, thienyl, pyrrolyl, oxazolyl, isoxazolyl, thiazolyl, isothiazolyl, imidazolyl, pyrazolyl, 1,2,3-oxadiazolyl, 1,2,4-oxadiazolyl, 1,3,4-oxadiazolyl, furazanyl, 1,2,3-thiadiazolyl, 1,2,4-thiadiazolyl, 1,3,4-thiadiazolyl, 1,2,3-triazolyl, 1,2,4-triazolyl, tetrazolyl, pyridyl, pyridazinyl, pyrimidinyl, pyrazinyl, triazinyl and the like; an aromatic fused heterocyclic group, such as benzofuranyl, isobenzofuranyl, benzo[b]thienyl, indolyl, isoindolyl, 1H-indazolyl, benzimidazolyl, benzoxazolyl, 1,2-benzisoxazolyl, benzothiazolyl, 1,2-benzisothiazolyl, 1H-benzotriazolyl, quinolyl, isoquinolyl, cinnolinyl, quinazolinyl, quinoxalinyl, phthalazinyl, naphthylidinyl, purinyl, pteridinyl, carbazolyl, α-carbolinyl, β-carbolinyl, γ-carbolinyl, acridinyl, phenoxazinyl, phenothiazinyl, phenazinyl, phenoxathiinyl, thianthrenyl, phenanthridinyl, phenanthrolinyl, indolizinyl, pyrrolo[1,2-b]pyridazinyl, pyrazolo[1,5-a]pyridyl, imidazo[1,2-a]pyridyl, imidazo[1,5-a]pyridyl, imidazo[1,2-b]pyridazinyl, imidazo[1,2-a]pyrimidinyl, 1,2,4-triazolo[4,3-a]pyridyl, 1,2,4-triazolo[4,3-b]pyridazinyl and the like, and the like.

Preferable examples of the non-aromatic heterocyclic group include oxiranyl, azetidinyl, oxetanyl, thietanyl, pyrrolidinyl, tetrahydrofuryl, thiolanyl, piperidyl, tetrahydropyranyl, morpholinyl, thiomorpholinyl, piperazinyl, pyrrolidino, piperidino, morpholino and the like.

Examples of the halogen atom include fluorine, chlorine, bromine and iodine.

In the optionally substituted amino group, the substituted amino group is exemplified by an N-monosubstituted amino group and an N,N-disubstituted amino group. As the substituted amino group, for example, an amino group having 1 or 2 substituents, selected from C₁₋₁₀ alkyl group, C₂₋₁₀ alkenyl group, C₂₋₁₀ alkynyl group, aromatic group, heterocyclic group and C₁₋₁₀ acyl group, is mentioned. Examples thereof include methylamino, dimethylamino, ethylamino, diethylamino, dibutylamino, diallylamino, cyclohexylamino, phenylamino, N-methyl-N-phenylamino, acetylamino, propionylamino, benzoylamino, nicotinoylamino and the like.

Examples of the acyl group include an acyl group having 1 to 13 carbon atoms, which is concretely an alkanoyl group having 1 to 10 carbon atoms, an alkenoyl group having 3 to 10 carbon atoms, a cycloalkanoyl group having 4 to 10 carbon atoms, a cycloalkenoyl group having 4 to 10 carbon atoms, an aromatic carbonyl group having 6 to 12 carbon atoms and the like.

Preferable examples of the alkanoyl group having 1 to 10 carbon atoms include formyl, acetyl, propionyl, butyryl, isobutyryl, valeryl, isovaleryl, pivaloyl, hexanoyl, heptanoyl, octanoyl and the like.

Preferable examples of the alkenoyl group having 3 to 10 carbon atoms include acryloyl, methacryloyl, crotonoyl, isocrotonoyl and the like.

Preferable examples of the cycloalkanoyl group having 4 to 10 carbon atoms include cyclobutanecarbonyl, cyclopentanecarbonyl, cyclohexanecarbonyl, cycloheptanecarbonyl and the like.

Preferable examples of the cycloalkenoyl group having 4 to 10 carbon atoms include 2-cyclohexenecarbonyl and the like.

Preferable examples of the aromatic carbonyl group having 6 to 12 carbon atoms include benzoyl, naphthoyl, nicotinoyl and the like.

The above-mentioned alkanoyl group, alkenoyl group, cycloalkanoyl group, cycloalkenoyl group and aromatic carbonyl group may have 1 to 3 substituents at optional substitutable positions. Examples of the substituent include an alkyl group having 1 to 3 carbon atoms, an alkoxy group having 1 to 3 carbon atoms, halogen atom (e.g., chlorine, fluorine, bromine etc.), a nitro group, a hydroxyl group, an amino group and the like.

In the optionally substituted hydroxyl group, examples of the substituted hydroxyl group include an alkoxy group, a cycloalkyloxy group, an alkenyloxy group, a cycloalkenyloxy group, an aralkyloxy group, an acyloxy group, an aryloxy group and the like.

Preferable examples of the alkoxy group include an alkoxy group having 1 to 10 carbon atoms, such as methoxy, ethoxy, propoxy, isopropoxy, butoxy, isobutoxy, sec.-butoxy, t.-butoxy, pentyloxy, isopentyloxy, neopentyloxy, hexyloxy, heptyloxy, nonyloxy and the like.

Preferable examples of the cycloalkyloxy group include a cycloalkyloxy group having 3 to 10 carbon atoms, such as cyclobutoxy, cyclopentyloxy, cyclohexyloxy and the like.

Preferable examples of the alkenyloxy group include an alkenyloxy group having 2 to 10 carbon atoms, such as allyloxy, crotyloxy, 2-pentenyloxy, 3-hexenyloxy and the like.

Preferable examples of the cycloalkenyloxy group include a cycloalkenyloxy group having 3 to 10 carbon atoms, such as 2-cyclopentenyloxy, 2-cyclohexenyloxy and the like.

Preferable examples of the aralkyloxy group include an aralkyloxy group having 7 to 10 carbon atoms, such as phenyl-C₁₋₄ alkyloxy (e.g., benzyloxy, phenethyloxy etc.) and the like.

Preferable examples of the acyloxy group include an acyloxy group having 2 to 13 carbon atoms, more preferably an alkanoyloxy having 2 to 4 carbon atoms (e.g., acetyloxy, propionyloxy, butyryloxy, isobutyryloxy etc.) and the like.

Preferable examples of the aryloxy group include an aryloxy group having 6 to 14 carbon atoms, such as phenoxy, naphthyloxy and the like. The aryloxy group may have 1 or 2 substituents. Examples of such substituent include a halogen atom (e.g., chlorine, fluorine, bromine etc.) and the like. Examples of the substituted aryloxy group include 4-chlorophenoxy and the like.

In the optionally substituted thiol group, examples of the substituted thiol group include an alkylthio group, a cycloalkylthio group, an alkenylthio group, a cycloalkenylthio group, an aralkylthio group, an acylthio group, an arylthio group and the like.

Preferable examples of the alkylthio group include an alkylthio group having 1 to 10 carbon atoms, such as methylthio, ethylthio, propylthio, isopropylthio, butylthio, isobutylthio, sec.-butylthio, t.-butylthio, pentylthio, isopentylthio, neopentylthio, hexylthio, heptylthio, nonylthio and the like.

Preferable examples of the cycloalkylthio group include a cycloalkylthio group having 3 to 10 carbon atoms, such as cyclobutylthio, cyclopentylthio, cyclohexylthio and the like.

Preferable examples of the alkenylthio group include an alkenylthio group having 2 to 10 carbon atoms, such as allylthio, crotylthio, 2-pentenylthio, 3-hexenylthio and the like.

Preferable examples of the cycloalkenylthio group include a cycloalkenylthio group having 3 to 10 carbon atoms, such as 2-cyclopentenylthio, 2-cyclohexenylthio and the like.

Preferable examples of the aralkylthio group include an aralkylthio group having 7 to 10 carbon atoms, such as phenyl-C₁₋₄ alkylthio (e.g., benzylthio, phenethylthio etc.) and the like.

Preferable examples of the acylthio group include an acylthio group having 2 to 13 carbon atoms, more preferably an alkanoylthio group having 2 to 4 carbon atoms (e.g., acetylthio, propionylthio, butyrylthio, isobutyrylthio etc.) and the like.

Preferable examples of the arylthio group include an arylthio group having 6 to 14 carbon atoms, such as phenylthio, naphthylthio and the like. The arylthio group may have 1 or 2 substituents. Examples of such substituent include a halogen atom (e.g., chlorine, fluorine, bromine etc.) and the like. Examples of the substituted arylthio group include 4-chlorophenylthio and the like.

Examples of the optionally esterified carboxyl group include an alkoxycarbonyl group, an aralkyloxycarbonyl group, an aryloxycarbonyl group and the like.

Preferable examples of the alkoxycarbonyl group include an alkoxycarbonyl group having 2 to 5 carbon atoms, such as methoxycarbonyl, ethoxycarbonyl, propoxycarbonyl, butoxycarbonyl and the like.

Preferable examples of the aralkyloxycarbonyl group include an aralkyloxycarbonyl group having 8 to 10 carbon atoms, such as benzyloxycarbonyl and the like.

Preferable examples of the aryloxycarbonyl group include an aryloxycarbonyl group having 7 to 15 carbon atoms, such as phenoxycarbonyl, p-tolyloxycarbonyl and the like.

The substituent of the hydrocarbon group and the heterocyclic group represented by R is preferably an alkyl group having 1 to 10 carbon atoms, an aromatic heterocyclic group and an aryl group having 6 to 14 carbon atoms, more preferably C₁₋₃ alkyl, furyl, thienyl, phenyl and naphthyl.

In the formula (I), the substituent of the hydrocarbon group and the heterocyclic group represented by R, when it is an alicyclic hydrocarbon group, an aryl group, an aromatic heterocyclic group or a non-aromatic heterocyclic group, may each have one or more, preferably 1 to 3, suitable additional substituents. Examples of such substituent include an alkyl group having 1 to 6 carbon atoms, an alkenyl group having 2 to 6 carbon atoms, an alkynyl group having 2 to 6 carbon atoms, a cycloalkyl group having 3 to 7 carbon atoms, an aryl group having 6 to 14 carbon atoms, an aromatic heterocyclic group (e.g., thienyl, furyl, pyridyl, oxazolyl, thiazolyl etc.), a non-aromatic heterocyclic group (e.g., tetrahydrofuryl, morpholino, thiomorpholino, piperidino, pyrrolidino, piperazino etc.), an aralkyl group having 7 to 9 carbon atoms, an amino group, an N-mono-C₁₋₄ alkylamino group, an N,N-di-C₁₋₄ alkylamino group, an acylamino group having 2 to 8 carbon atoms (e.g., acetylamino, propionylamino, benzoylamino etc.), an amidino group, an acyl group having 2 to 8 carbon atoms (e.g., alkanoyl group having 2 to 8 carbon atoms etc.), a carbamoyl group, an N-mono-C₁₋₄ alkylcarbamoyl group, an N,N-di-C₁₋₄ alkylcarbamoyl group, a sulfamoyl group, an N-mono-C₁₋₄ alkylsulfamoyl group, an N,N-di-C₁₋₄ alkylsulfamoyl group, a carboxyl group, an alkoxycarbonyl group having 2 to 8 carbon atoms, a hydroxyl group, an alkoxy group having 1 to 4 carbon atoms, an alkenyloxy group having 2 to 5 carbon atoms, a cycloalkyloxy group having 3 to 7 carbon atoms, an aralkyloxy group having 7 to 9 carbon atoms, an aryloxy group having 6 to 14 carbon atoms, a mercapto group, an alkylthio group having 1 to 4 carbon atoms, an aralkylthio group having 7 to 9 carbon atoms, an arylthio group having 6 to 14 carbon atoms, a sulfo group, a cyano group, an azide group, a nitro group, a nitroso group, a halogen atom and the like.

In the formula (I), R is preferably an optionally substituted heterocyclic group. R is more preferably pyridyl, oxazolyl or thiazolyl group each optionally having 1 to 3 substituents selected from C₁₋₃ alkyl, furyl, thienyl, phenyl and naphthyl.

In the formula (I), Y is -CO-, -CH(OH)- or -NR³- (R³ is an optionally substituted alkyl group), preferably -CH(OH)- or -NR³-. As used herein, examples of the alkyl group of the optionally substituted alkyl group, which is represented by R³, include, an alkyl group having 1 to 4 carbon atoms, such as methyl, ethyl, propyl, isopropyl, butyl, isobutyl, sec.-butyl, t.-butyl and the like. Examples of the substituent include a halogen atom (e.g., fluorine, chlorine, bromine and iodine), an alkoxy group having 1 to 4 carbon atoms (e.g., methoxy, ethoxy, propoxy, butoxy, isobutoxy, sec.-butoxy, t.-butoxy etc.), a hydroxyl group, a nitro group and an acyl group having 1 to 4 carbon atoms (e.g., formyl, acetyl, propionyl etc.).
m is 0 or 1, preferably 0.
n is 0, 1 or 2, preferably 0 or 1.
X is CH or N, preferably CH.

In the formula (I), A is a bond or a divalent aliphatic hydrocarbon group. The aliphatic hydrocarbon group may be straight-chain or branched and saturated or unsaturated. The divalent aliphatic hydrocarbon group is preferably a divalent aliphatic hydrocarbon group having 1 to 7 carbon atoms, which is, for example, a saturated one such as -CH₂-, -CH(CH₃)-, -(CH₂)₂-, -CH(C₂H₅)-, -(CH₂)₃-, -(CH₂)₄-, -(CH₂)₅-, -(CH₂)₆-, -(CH₂)₇- and the like, an unsaturated one such as -CH=CH-, -C(CH₃)=CH-, -CH=CH-CH₂-, -C(C₂H₅)=CH-, -CH₂-CH=CH-CH₂-, -CH₂-CH₂-CH=CH-CH₂-, -CH=CH-CH=CH-CH₂-, -CH=CH-CH=CH-CH=CH-CH₂- and the like. A is preferably a bond or a divalent aliphatic hydrocarbon group having 1 to 4 carbon atoms, and the aliphatic hydrocarbon group is preferably saturated. A is more preferably a bond or -(CH₂)₂-.

As the alkyl group represented by R¹, those exemplified for the alkyl group of the aforementioned R³ may be used. R¹ is preferably a hydrogen atom.

In the formula (I), the partial structural formula 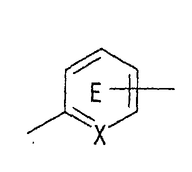 preferably represents the formula 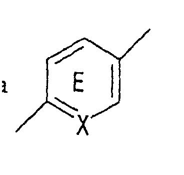 wherein each symbol is as defined above.

The ring E optionally has additional substituents at optional substitutable positions. Examples of such substituent include an alkyl group, an optionally substituted hydroxyl group, a halogen atom, an acyl group and an optionally substituted amino group. As these, those exemplified for the substituents of the hydrocarbon group and heterocyclic group represented by the aforementioned R are used. The number of the substituent is preferably 1 to 4.

The ring E, or a partial structural formula 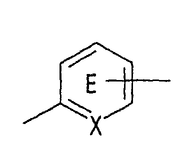 is preferably the formula 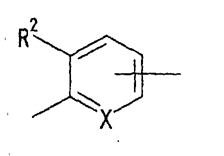 wherein R² is a hydrogen atom, an alkyl group, an optionally substituted hydroxyl group, a halogen atom, an acyl group, a nitro group or an optionally substituted amino group.

The alkyl group, optionally substituted hydroxyl group, halogen atom, acyl group, and optionally substituted amino group represented by R² are exemplified by those exemplified for the substituents of the hydrocarbon group and heterocyclic group of the aforementioned R. R² is preferably a hydrogen atom, an optionally substituted hydroxyl group or a halogen atom. R² is more preferably a hydrogen atom or an optionally substituted hydroxyl group, particularly preferably a hydrogen atom or an alkoxyl group having 1 to 4 carbon atom.

In the formula (I), L and M are each a hydrogen atom or bonded to each other to show a bond, preferably a hydrogen atom.

The compound wherein L and M are bonded to each other to form a bond contains an (E) form and a (Z) form due to the double bond at the 5-position of the azolidinedione ring.

The compound wherein L and M are each a hydrogen atom contains optical isomers of an (R)-form and an (S)-form due to the asymmetric carbon at the 5-position of the azolidinedione ring, and this compound contains an optically active form and racemates of these (R)-form and (S)-form.

Preferable examples of the compound represented by the formula (I) include a compound wherein R is pyridyl, oxazolyl or thiazolyl group optionally having 1 to 3 substituents selected from C₁₋₃ alkyl, furyl, thienyl, phenyl and naphthyl; m is 0; n is 0 or 1; X is CH; A is a bond or -(CH₂)₂-; R¹ is a hydrogen atom; ring E or a partial structural formula 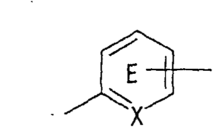 is the formula 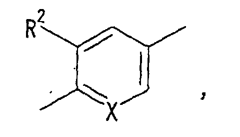 R² is a hydrogen atom or a C₁₋₄ alkoxy group; and L and M are hydrogen atoms.

Preferable examples of the compound represented by the formula (I) include the following compounds (A)-(D) and the like.
(A) 5-[4-[2-(5-ethyl-2-pyridyl)ethoxy]benzyl]-2,4-thiazolidinedione (general name: pioglitazone);
(B) 5[[4-[(3,4-dihydro-6-hydroxy-2,5,7,8-tetramethyl-2H-1-benzopyran-2-yl)methoxy]phenyl]methyl]-2,4-thiazolidinedione (general name: troglitazone);
(C) 5-[[4-[2-(methyl-2-pyridinylamino)ethoxy]phenyl]methyl]-2,4-thiazolidinedione (general name: rosiglitazone);
(D) (R)-(+)-5-[3-[4-[(5-methyl-2-phenyl-4-thiazolyl)methoxy]phenyl]propyl]-2,4-oxazolidinedione (described in Japanese Patent Application No. 2000-24773); and the like.

These compounds are hereinafter sometimes to be simply referred to as compound (A), compound (B) and the like.

The compound represented by the formula (I) is particularly preferably pioglitazone.

The salt of the compound represented by the formula (I) includes a pharmacologically acceptable salt, such as a salt with an inorganic base, a salt with an organic base, a salt with an inorganic acid, a salt with an organic acid, a salt with a basic or acidic amino acid and the like.

Preferable examples of the salt with an inorganic base include, a salt with an alkali metal such as sodium, potassium and the like, an alkaline earth metal such as calcium, magnesium and the like, and a salt with aluminum, ammonium and the like.

Preferable examples of the salt with an organic base include a salt with trimethylamine, triethylamine, pyridine, picoline, ethanolamine, diethanolamine, triethanolamine, dicyclohexylamine, N,N-dibenzylethylenediamine and the like.

Preferable examples of the salt with an inorganic acid include a salt with hydrochloric acid, hydrobromic acid, nitric acid, sulfuric acid, phosphoric acid and the like.

Preferable examples of the salt with an organic acid include a salt with formic acid, acetic acid, trifluoroacetic acid, fumaric acid, oxalic acid, tartaric acid, maleic acid, citric acid, succinic acid, malic acid, methanesulfonic acid, benzenesulfonic acid, p-toluenesulfonic acid and the like.

Preferable examples of the salt with a basic amino acid include a salt with arginine, lysin, ornithine and the like, and preferable examples of the salt with an acidic amino acid include a salt with aspartic acid, glutamic acid and the like.

The compound represented by the formula (I) or a salt thereof is preferably pioglitazone hydrochloride, troglitazone or rosiglitazone, particularly preferably pioglitazone hydrochloride.

The compound represented by the formula (I) or a salt thereof can be produced by, for example, a method described in JP-A-55-22636 (EP-A 8203), JP-A-60-208980 (EP-A 155845), JP-A-61-286376 (EP-A 208420), JP-A-61-85372 (EP-A 177353), JP-A-61-267580 (EP-A 193256), JP-A-5-86057 (WO 92/18501), JP-A-7-82269 (EP-A 605228), JP-A-7-101945 (EP-A 612743), EP-A-643050, EP-A-710659 and the like, or a method analogous thereto.

The "insulin sensitizer other than compound (II)" is exemplified by, besides those mentioned above,
4-[4-[2-(5-methyl-2-phenyloxazol-4-yl)ethoxy]benzyl] isoxazolidine-3,5-dione (JTT-501);
5-[[3,4-dihydro-2-(phenylmethyl)-2H-1-benzopyran-6-yl]methyl]-2,4-thiazolidinedione (general name: englitazone);
5-[[4-[3-(5-methyl-2-phenyl-4-oxazolyl)-1-oxopropyl]phenyl]methyl]-2,4-thiazolidinedione (general name: darglitazone/CP-86325);
5-[2-(5-methyl-2-phenyl-4-oxazolylmethyl)benzofuran-5-ylmethyl]-2,4-oxazolidinedione (CP-92768);
5-(2-naphthalenylsulfonyl)-2,4-thiazolidinedione (AY-31637);
4-[(2-naphthalenyl)methyl]-3H-1,2,3,5-oxathiadiazole-2-oxide (AY-30711);
5-[[6-(2-fluorobenzyloxy)-2-naphthyl]methyl]-2,4-thiazolidinedione (MCC-555);
[5-[(2,4-dioxothiazolidin-5-yl)methyl]-2-methoxy-N-[[4-(trifluoromethyl)phenyl]methyl]benzamide (AHG-255);
4-[1-(3,5,5,8,8-pentamethyl-5,6,7,8-tetrahydronaphthalen-2-yl)ethenyl]benzoic acid (LGD1069);
6-[1-(3,5,5,8,8-pentamethyl-5,6,7,8-tetrahydronaphthalen-2-yl)cyclopropyl]nicotinic acid (LG100268);
1,4-bis[4-[(3,5-dioxo-1,2,4-oxadiazolidin-2-yl)methyl]phenoxy]-2-butene (YM-440);
bexarotene; GI-262570; DRF-2593; HQL-975; DN-108; CS-011; dexlipotam; INS-1; AR-H-0329242; CLX-0901; FK-614; KRP-297; CRE-16336; NN-2344; BM-13-1258; S-15261; KB-R-7785; DRF-2725; GW-2570; GW-2433; MXC-3255; L-746449; L-767827; L-783281; GW-409544 and the like.

The above-mentioned compound may be used as a salt. As such salt, those similar to the salts of the compound represented by the aforementioned formula (I) are used.

The "insulin sensitizer other than compound (II)" is preferably pioglitazone (preferably hydrochloride thereof), troglitazone, rosiglitazone (preferably maleate thereof) and the like.

The aforementioned "insulin sensitizer other than compound (II)" may be used upon combination of two or more kinds thereof at a suitable ratio.

The angiogenesis inhibitor of the present invention may be compound (II) itself, which is an active ingredient, but generally, it is produced by admixing the active ingredient with a pharmacologically acceptable carrier according to a method known *per se* [conventional method in the technical field of pharmaceutical manufacturing engineering, such as the method described in The Japanese Pharmacopoeia (e.g., JP XIII) and the like].

Examples of the dosage form of the angiogenesis inhibitor of the present invention include an oral preparations such as tablet, capsule (including soft capsule and microcapsule), powder, granule, syrup and the like; and a parenteral preparations such as injection (e.g., subcutaneous injection, intravenous injection, intramuscular injection, intraperitoneal injection etc.), external preparation (e.g., preparation for nasal administration, percutaneous preparation, ointment etc.), suppository (e.g., rectal suppository, vaginal suppository etc.), pellet, drops, sustained-release preparation (e.g., sustained-release microcapsule etc.) and the like.

The production methods of oral preparations and parenteral preparations are explained in detail in the following.

An oral preparation can be produced by adding, to the active ingredient, for example, an excipient (e.g., lactose, saccharose, starch, D-mannitol, xylitol, sorbitol, erythritol, microcrystalline cellulose, light silicic anhydride etc.), a disintegrant (e.g., calcium carbonate, starch, carboxymethyl cellulose, carboxymethyl cellulose calcium, low-substituted hydroxypropylcellulose, croscarmellose sodium, carboxymethyl starch sodium, light silicic anhydride etc.), a binder (e.g., pregelatinized starch, gum arabic, carboxymethyl cellulose, hydroxypropylcellulose, hydroxypropylmethylcellulose, polyvinylpyrrolidone, microcrystalline cellulose, methyl cellulose, saccharose, D-mannitol, trehalose, dextrin etc.), a lubricant (e.g., talc, magnesium stearate, calcium stearate, colloidal silica, polyethylene glycol 6000 etc.) and the like, and compression-molding the mixture. To the oral preparation, an acid such as hydrochloric acid, phosphoric acid, malonic acid, succinic acid, DL-malic acid, tartaric acid, maleic acid, fumaric acid, citric acid and the like or a base such as sodium carbonate, sodium hydrogencarbonate, sodium citrate, sodium tartrate and the like may be added for promoting dissolution of the active ingredients.

Furthermore, the oral preparation may be coated by a method known *per se* for the purpose of masking a taste, enteric coating, or achieving a sustained release. Examples of the coating agent include an enteric polymer (e.g., cellulose acetate phthalate, methacrylic acid copolymer L, methacrylic acid copolymer LD, methacrylic acid copolymer S, hydroxypropylmethylcellulose phthalate, hydroxypropylmethylcellulose acetate succinate, carboxymethylethylcellulose etc.), a gastrosoluble polymer (e.g., polyvinylacetal diethylaminoacetate, aminoalkyl methacrylate copolymer E etc.), a water-soluble polymer (e.g., hydroxypropylcellulose, hydroxypropylmethylcellulose etc.), a water-insoluble polymer (e.g., ethylcellulose, aminoalkyl methacrylate copolymer RS, ethyl acrylate/methyl methacrylate copolymer etc.), wax and the like. For coating, a plasticizer such as polyethylene glycol etc. and a shading agent such as titanium oxide, iron sesquioxide etc. may be used, along with the above-mentioned coating agents.

An injection can be produced by dissolving, suspending or emulsifying an active ingredient in an aqueous solvent (e.g., distilled water, physiological saline, Ringer's solution etc.), an oily solvent (e.g., vegetable oil such as olive oil, sesame oil, cottonseed oil, corn oil etc.; propylene glycol, macrogol, tricaprylin etc.) and the like, together with a dispersant [e.g., Tween 80 (Atlas Powder Co., U.S.A.), HCO 60 (Nikko Chemicals Co., Ltd.), polyethylene glycol, carboxymethyl cellulose, sodium alginate etc.], a preservative (e.g., methyl paraben, propyl paraben, benzyl alcohol, chlorobutanol, phenol etc.), an isotonizing agent (e.g., sodium chloride, glycerine, D-sorbitol, D-mannitol, xylitol, glucose, fructose etc.) and the like.

Where desired, additives may be used, such as a dissolution aid (e.g., sodium salicylate, sodium acetate, polyethylene glycol, propylene glycol, D-mannitol, trehalose, benzyl benzoate, ethanol, Tris aminomethane, cholesterol, triethanolamine, sodium carbonate, sodium citrate etc.), a suspending agent (e.g., surfactant such as stearyltriethanolamine, sodium lauryl sulfate, laurylaminopropionic acid, lecithin, benzalkonium chloride, benzethonium chloride, glycerine monostearate etc.); a hydrophilic polymer such as polyvinyl alcohol, polyvinylpyrrolidone, carboxymethylcellulose sodium, methylcellulose, hydroxymethylcellulose, hydroxyethylcellulose, hydroxypropylcellulose etc.), a buffering agent (e.g., buffer of phosphate, acetate, carbonate, citrate etc.), a stabilizer (e.g., human serum albumin etc.), a soothing agent (e.g., propylene glycol, lidocaine hydrochloride, benzyl alcohol etc.), a preservative (e.g., p-oxybenzoic acid esters, chlorobutanol, benzalkonium chloride, benzyl alcohol, phenethyl alcohol, dehydroacetic acid, sorbic acid etc.), and the like.

An external preparation can be produced by forming an active ingredient into a solid, semisolid or liquid composition. For example, the above-mentioned solid composition can be produced by making a powder of an active ingredient as it is or upon addition of and mixing with an excipient (e.g., lactose, D-mannitol, starch, crystalline cellulose, saccharose etc.), a thickening agent (e.g., natural gum, cellulose derivative, acrylate polymer etc.) and the like. The above-mentioned liquid composition can be produced in almost the same manner as in the case of injections. A semisolid composition is preferably an aqueous or oily gel, or an ointment. These compositions may contain a pH adjuster (e.g., phosphoric acid, citric acid, hydrochloric acid, sodium hydroxide etc.), a preservative (e.g., p-oxybenzoic acid esters, chlorobutanol, benzalkonium chloride, benzyl alcohol, phenethyl alcohol, dehydroacetic acid, sorbic acid etc.), and the like.

A suppository is produced by forming an active ingredient into an oily or aqueous solid, semisolid or liquid composition. As an oily base to be used for the production of the composition may be, for example, higher fatty acid glyceride [e.g., cacao butter, witepsols (Huels Aktiengesellschaft, Germany) etc.], a medium chain fatty acid triglyceride [e.g., Migriols (Huels Aktiengesellschaft, Germany) etc.], a vegetable oil (e.g., sesame oil, soybean oil, cottonseed oil etc.), and the like. As an aqueous base, for example, polyethylene glycols, propylene glycol and the like are mentioned. As an aqueous gel base, for example, natural gum, cellulose derivative, vinyl polymer, acrylic acid polymer and the like are mentioned.

The content of compound (II) in the angiogenesis inhibitor of the present invention is, for example, 0.1 to 100 wt%, preferably 5 to 80 wt%.

The angiogenesis inhibitor of the present invention is low in toxicity and can be administered safely to a mammal (e.g., human, mouse, rat, rabbit, dog, cat, cattle, horse, pig, monkey etc.) orally or parenterally.

The dose of the angiogenesis inhibitor of the present invention follows the dose of compound (II), which is the active ingredient, and can be appropriately determined depending on the administration subject, age and body weight of the administration subject, symptom, administration time, dosage form, administration method and the like. The dose of compound (II) can be appropriately determined based on the clinically employed dose.

For example, when an angiogenesis inhibitor is administered to an adult (body weight 50 kg), the daily dose in terms of compound (II) as the active ingredient is generally 0.01-1000 mg, preferably 0.1-600 mg, more preferably 10-200 mg, and this amount can be administered once or several times a day in divided portions.

When compound (II) and an "insulin sensitizer other than compound (II)" is used in combination, the dose of the "insulin sensitizer other than compound (II)" can be appropriately determined based on the clinically employed dose.

For example, when an "insulin sensitizer other than compound (II)" is administered to an adult (body weight 50 kg), the daily dose is generally 0.01-1000 mg, preferably 0.1-600 mg, and this amount can be administered once or several times a day in divided portions.

When pioglitazone hydrochloride is orally administered to an adult (body weight 50 kg) as the "insulin sensitizer other than compound (II)", the daily dose is generally 7.5 - 60 mg, preferably 15 - 45 mg, and this amount can be administered once or twice a day in divided portions.

When troglitazone is orally administered to an adult (body weight 50 kg) as the "insulin sensitizer other than compound (II)", the daily dose is generally 100- 1000 mg, preferably 200 - 600 mg, and this amount can be administered once or twice a day in divided portions.

When rosiglitazone (or maleate thereof) is orally administered to an adult (body weight 50 kg) as the "insulin sensitizer other than compound (II)", the daily dose is generally 1 - 12 mg, preferably 2 - 12 mg, and this amount can be administered once or twice a day in divided portions.

The angiogenesis inhibitor of the present invention has a superior angiogenesis (growth of vascular endothelial cell) inhibitory action. As used herein, the angiogenesis may be induced by, for example, a cell growth factor such as vascular endothelial growth factor (VEGF), basic fibroblast growth factor (bFGF) and the like. The angiogenesis inhibitor of the present invention can exert an angiogenesis inhibitory action by suppressing the expression or action of these cell growth factors or without influencing the cell growth factors.

Therefore, the angiogenesis inhibitor of the present invention can be used as an agent for the prophylaxis or treatment of various diseases associated with angiogenesis, such as tumor [e.g., malignant melanoma, malignant lymphoma, cancer of digestive organ (e.g., stomach, intestine etc.), lung cancer, pancreatic cancer, esophageal cancer, breast cancer, liver cancer, ovarian cancer, uterine cancer, prostate cancer, renal cancer, bladder cancer, brain tumor, Kaposi's sarcoma, angioma, osteogenic sarcoma, myosarcoma, angiofibroma etc.], inflammatory disease (e.g., rheumarthritis, psoriasis etc.), diabetic retinopathy, atherosclerosis (including abnormal neovascularization in abnormal capillary plexus formation in outeratheromatous membrane of atheromatous arteriosclerosis) and the like. In addition, the angiogenesis inhibitor of the present invention is used as a therapeutic agent of hyperemia of the eye.

In the angiogenesis inhibitor of the present invention, it is possible to use a pharmaceutical agent for combined use, which does not exert an adverse influence on the angiogenesis inhibitory action of compound (II), with the aim of "potentiation of angiogenesis inhibitory action of compound (II)", "reduction of amount of compound (II) to be used", "reduction of side effects of compound (II)" and the like. Examples of such pharmaceutical agent for combined use include "an antitumor drug", "a cachexia-improving drug", "a therapeutic drug of diabetes other than insulin sensitizer", "a therapeutic drug of diabetic complications", "an anti-obesity drug", "a therapeutic drug of hypertension", "a therapeutic drug of hyperlipidemia", "a diuretic agent" and the like. In addition, a surgery (operation) or a radiotherapy may be given when using the angiogenesis inhibitor of the present invention.

Examples of the above-mentioned "antitumor drug" include an angiogenesis inhibitor (e.g., thalidomide, rinomastat, endostatin, angiostatin, carbamidetriazole, replistatin, 6-o-(N-chloroacetylcarbamoyl)fumagillol, integrin αvβ3 inhibitor etc.), anti-estrogen agent (e.g., tamoxifen, raloxifene, droloxifen, idoxifen etc.), progestogens (e.g., megestol acetate etc.), aromatase inhibitor (e.g., anastrozole, letrazole, volozole, exemestane etc.), anti-progestogen agent, anti-androgen agent (e.g., flutamide, niltamide, bicalutamide, cyproterone acetate etc.), LHRH agonist or antagonist (e.g., leapronde, leuprorelin acetate, goserelin acetate, cetrorelix, abarelix etc.), testosterone 5α-reductase inhibitor (e.g., finasteride etc.), metalloproteinase inhibitor (e.g., marimastat etc.), urokinase plasminogen activator receptor inhibitor, antibody against growth factor (e.g., EGF, FGF, PDGF, HGF etc.), antibody against growth factor (e.g., EGF, FGF, PDGF, HGF etc.) receptor, tyrosine kinase inhibitor, serine/threonine kinase inhibitor, antifolic (e.g., methotrexate etc.), fluoropyrimidine derivative (e.g., 5-fluorouracil, furtulon, neofurtulon etc.), antitumor antibiotics (e.g., doxorubicin, daunomycin, epirubicin, idarubicin, mitomycin-C, dactinomycin, mithramycin, adriamycin etc.), platinum derivatives (e.g., cisplatin, carboplatin etc.), alkylating agent (e.g., nitrogen mustards, melphalan, chlorambucin, busulfan, cyclophosphamide, nitrosoureas, thiotepa etc.), vinca alkaloids (e.g., vincristine etc.), taxol or its derivatives (e.g., taxol, taxotere etc.), podophyllotoxins (e.g., etoposide, teniposide etc.), topoisomerase inhibitor (e.g., amsacrine, topotecan, irinotecan etc.), microorganisms or bacteria-derived immunotherapy agents (e.g., muramyl dipeptide derivative, picibanil etc), polysaccharides having immunity-potentiating activity (e.g., lenthinan, schizophyllan, krestin etc.), cytokine [e.g., interferon, interleukins (preferably IL-1, IL-2, IL-12 etc.), colony stimulating factor (e.g., granulocyte-colony stimulating factor, erythropoietin etc.) and the like.

Examples of the above-mentioned "cachexia-improving drug" include cyclooxygenase inhibitor (e.g., indomethacin etc.), progesterone derivative (e.g., megestrol acetate), glucosteroid (e.g., dexamethasone etc.), metoclopramide agent, tetrahydrocannabinol agent, fat metabolism improving agent (e.g., eicosapentaenoic acid etc.), growth hormone, IGF-1, and antibodies against cachexia inducing factor (e.g., TNF-α, LIF, IL-6, oncostatin M etc.) and the like.

The above-mentioned "therapeutic drug of diabetes other than insulin sensitizer" is, for example, insulin secretagogue, biguanide, insulin, α-glucosidase inhibitor, β3 agonist and the like.

The insulin secretagogues include, for example, a sulfonylurea agent. Specific examples of the sulfonylurea agent include tolbutamide, chlorpropamide, tolazamide, acetohexamide, glyclopyramide and its ammonium salt, glibenclamide, gliclazide, 1-butyl-3-metanilylurea, carbutamide, glibornuride, glipizide, gliquidone, glisoxepide, glybuthiazole, glybuzole, glyhexamide, glymidine, glypinamide, phenbutamide, tolcyclamide, glimepiride and the like.

Besides the above-mentioned, the insulin secretagogue is exemplified by N-[[4-(1-methylethyl)cyclohexyl]carbonyl]-D-phenylalanine (Nateglinide, AY-4166), calcium (2S)-2-benzyl-3-(cis-hexahydro-2-isoindolinylcarbonyl)propionate dihydrate (Mitiglinide), KAD-1229], Repaglinide, GLP(Glucagon-like peptide)-1, GLP-1(7-36)amide, V8-GLP-1 (LY-307161), Exendin-4 (AC-2993), DPP-728-A, V-411, JT-608 and the like.

The biguanides are exemplified by phenformin, metformin, buformin and the like.

Examples of insulins, animal insulin extracted from the pancreas of cattle or pig; semi-synthetic human insulin enzymatically synthesized from insulin extracted from the pancreas of pig; human insulin genetically engineered *using E. coli* and yeast; and the like. As the insulin, zinc insulin containing 0.45 - 0.9 (w/w)% of zinc; protamine zinc insulin produced from zinc chloride, protamine sulfate and insulin, and the like can be also used. The insulin may be a fragment or a derivative (e.g., INS-1 etc.) thereof.

The insulin includes various kinds such as ultra fast-acting type, fast-acting type, biphasic type, intermediate-acting type, long-acting type and the like. These can be selected as appropriate depending on the disease state of the patient.

Examples of α-glucosidase inhibitors include acarbose, voglibose, miglitol, emiglitate and the like.

Examples of the β3 agonists (β3 adrenaline receptor agonist) include AJ-9677, SR-58611-A, SB-226552 and the like.

Besides the above-mentioned, the "therapeutic drug of diabetes other than insulin sensitizer" includes, for example, ergoset, pramlintide, leptin, BAY-27-9955, T-1095 and the like.

Examples of the aforementioned "therapeutic drug of diabetic complications" include an aldose reductase inhibitor, a glycation inhibitor, a protein kinase C inhibitor and the like.

Examples of the aldose reductase inhibitor include tolrestat; epalrestat; 3,4-dihydro-2,8-diisopropyl-3-thioxo-2H-1,4-benzoxazine-4-acetic acid; imirestat; zenarestat; 6-fluoro-2,3-dihydro-2',5'-dioxo-spiro[4H-1-benzopyran-4,4'-imidazolidine]-2-carboxamide (SNK-860); zopolrestat; sorbinil; and 1-[(3-bromo-2-benzofuranyl)sulfonyl]-2,4-imidazolidinedione (M-16209); CT-112; NZ-314; ARI-509 and the like.

Examples of the glycation inhibitor include pimagedine and the like.

Examples of the protein kinase C inhibitor include NGF, LY-333531 and the like.

Besides the above-mentioned, examples of the "therapeutic drug of diabetic complications" include alprostadil, tiapride hydrochloride, cilostazol, mexiletine hydrochloride, ethyl icosapentate, memantine, pimagedline (ALT-711) and the like.

Examples of the aforementioned "anti-obesity drug" include a lipase inhibitor, an anorectic drug and the like.

Examples of the lipase inhibitor include orlistat and the like.

Examples of the anorectic drug include dexfenfluramine, fluoxetine, sibutramine, biamine and the like.

Examples of the aforementioned "therapeutic drug of hypertension" include an angiotensin converting enzyme inhibitor, a calcium antagonist, a potassium channel opener, angiotensin II antagonists and the like.

Examples of the angiotensin converting enzyme inhibitor include captopril, enalapril, alacepril, delapril, ramipril, lisinopril, imidapril, benazepril, ceronapril, cilazapril, enalaprilat, fosinopril, moveltopril, perindopril, quinapril, spirapril, temocapril, trandolapril, manidipine and the like.

Examples of the calcium antagonist include nifedipine, amlodipine, efonidipine, nicardipine and the like.

Examples of the potassium channel opener include levcromakalim, L-27152, AL 0671, NIP-121 and the like.

Examples of the angiotensin II antagonist include losartan, candesartan cilexetil, valsartan, irbesartan, (5-methyl-2-oxo-1,3-dioxolen-4-yl)methyl 4-(1-hydroxy-1-methylethyl)-2-propyl-1-[2'-(1H-tetrazol-5-yl)biphenyl-4-ylmethyl]imidazole-5-carboxylate (CS-866), E4177 and the like.

Examples of the aforementioned "therapeutic drug of hyperlipidemia" include HMG-CoA reductase inhibitors, a fibrate compound and the like.

Examples of the HMG-CoA reductase inhibitors include pravastatin, simvatatin, lovastatin, atorvastatin, fluvastatin, lipantil, cerivastatin, itavastatin, ZD-4522 and salts thereof (e.g., sodium salt) and the like.

Examples of the fibrate compound include bezafibrate, beclobrate, binifibrate, ciprofibrate, clinofibrate, clofibrate, clofibrinic acid, etofibrate, fenofibrate, gemfibrozil, nicofibrate, pirifibrate, ronifibrate, simfibrate, theofibrate and the like.

Examples of the aforementioned "diuretic agent" include a xanthine derivative preparation, a thiazide preparation, an anti-aldosterone preparation, a carbonic anhydrase inhibitor, a chlorobenzenesulfonamide preparation and the like.

Examples of the xanthine derivative preparation include theobromine sodium salicylate, theobromine calcium salicylate and the like.

Examples of the thiazide preparation include ethiazide, cyclopenthiazide, trichlormethiazide, hydrochlorothiazide, hydroflumethiazide, benzylhydrochlorothiazide, penflutizide, polythiazide, methyclothiazide and the like.

Examples of the anti-aldosterone preparation include spironolactone, triamterene and the like.

Examples of the carbonic anhydrase inhibitor include acetazolamide and the like.

Examples of the chlorobenzenesulfonamide preparation include chlorthalidone, mefruside, indapamide and the like.

Besides the above-mentioned, examples of the "diuretic agent" include azosemide, isosorbide, ethacrynic acid, piretanide, bumetanide, furosemide and the like.

The aforementioned pharmaceutical agents for combined use may be used in combination of two or more optional agents thereof. Specific exemplary combinations when two kinds of pharmaceutical agents for combined use are to be used in combination include "a combination of an insulin secretagogue and a biguanide", "a combination of an insulin secretagogue and an α-glucosidase inhibitor", "a combination of insulin and a biguanide", "a combination of insulin and an α-glucosidase inhibitor" and the like.

The timing of the administration of the compound (II) and the pharmaceutical agent for combined use is not limited. These may be simultaneously administered to an administration subject or administered in a staggered manner. The dose of the pharmaceutical agent for combined use can be determined as appropriate by referring to the dose clinically employed, and depending on the administration subject, age and body weight of administration subject, symptom, administration time, dosage form, administration method, combination and the like.

The mode of administration of the pharmaceutical agent for combined use is not particularly limited, as long as compound (II) and a pharmaceutical agent for combined use are combined on administration. Examples of such administration mode include (1) administration of a single preparation obtained by simultaneous formulation of compound (II) and the pharmaceutical agent for combined use, (2) simultaneous administration by the same administration route of two kinds of preparations obtained by separate formulation of compound (II) and the pharmaceutical agent for combined use, (3) staggered administration by the same administration route of two kinds of preparations obtained by separate formulation of compound (II) and the pharmaceutical agent for combined use, (4) simultaneous administration by different administration routes of two kinds of preparations obtained by separate formulation of compound (II) and the pharmaceutical agent for combined use, (5) staggered administration by different administration routes of two kinds of preparations obtained by separate formulation of compound (II) and the pharmaceutical agent for combined use, such as administration in the order of compound (II) and then the pharmaceutical agent for combined use, or in the reversed order, and the like.

For example, when the "therapeutic drug of diabetes other than insulin sensitizer" is administered to an adult (body weight 50 kg), the daily dose is generally 0.1-2500 mg, preferably 0.5-1000 mg, and this amount can be administered once or several times a day in divided portions.

When an insulin secretagogue is administered to an adult (body weight 50 kg), the daily dose is generally 0.1-1000 mg, preferably 1-100 mg, and this amount can be administered once or several times a day in divided portions.

When a biguanide is administered to an adult (body weight 50 kg), the daily dose is generally 10-2500 mg, preferably 100-1000 mg, and this amount can be administered once or several times a day in divided portions.

When insulin is administered (can be administered as an injection, an oral agent, a nasal agent, a transpulmonary agent etc., but generally administered as an injection) to an adult (body weight 50 kg), the daily dose is generally 10-100U (units), preferably 10-80U (units), and this amount can be administered once or several times a day in divided portions.

When an α-glucosidase inhibitor is administered to an adult (body weight 50 kg), the daily dose is generally 0.1-400 mg, preferably 0.6-300 mg, and this amount can be administered once or several times a day in divided portions.

When a β3 agonist is administered to an adult (body weight 50 kg), the daily dose is generally 10-2000 mg, preferably 100-1000 mg, and this amount can be administered once or several times a day in divided portions.

When a "therapeutic drug of diabetic complications" is administered to an adult (body weight 50 kg), the daily dose is generally 0.1-2000 mg, and this amount can be administered once or several times a day in divided portions.

When an aldose reductase inhibitor is administered to an adult (body weight 50 kg), the daily dose is generally 1-1000 mg, and this amount can be administered once or several times a day in divided portions.

When a glycation inhibitor is administered to an adult (body weight 50 kg), the daily dose is generally 1-2000 mg, and this amount can be administered once or several times a day in divided portions.

When a protein kinase C inhibitor is administered to an adult (body weight 50 kg), the daily dose is generally 0.1-100 mg, and this amount can be administered once or several times a day in divided portions.

When, for example, an "anti-obesity drug" is administered to an adult (body weight 50 kg), the daily dose is generally 0.01-1000 mg, preferably 0.1-1000 mg, and this amount can be administered once or several times a day in divided portions.

When a lipase inhibitor is administered to an adult (body weight 50 kg), the daily dose is generally 0.1-1000 mg, and this amount can be administered once or several times a day in divided portions.

When an anorectic drug is administered to an adult (body weight 50 kg), the daily dose is generally 0.01-1000 mg, preferably 0.1-500 mg, and this amount can be administered once or several times a day in divided portions.

When a "therapeutic drug of hypertension" is administered to an adult (body weight 50 kg), the daily dose is generally 0.01-1000 mg, and this amount can be administered once or several times a day in divided portions.

When an angiotensin converting enzyme inhibitor is administered to an adult (body weight 50 kg), the daily dose is generally 0.01-500 mg, preferably 0.1-100 mg, and this amount can be administered once or several times a day in divided portions.

When a calcium antagonist is administered to an adult (body weight 50 kg), the daily dose is generally 0.1-500 mg, preferably 1-200 mg, and this amount can be administered once or several times a day in divided portions.

When a potassium channel opner is administered to an adult (body weight 50 kg), the daily dose is generally 0.01-1000 mg, and this amount can be administered once or several times a day in divided portions.

When an angiotensin II antagonist is administered to an adult (body weight 50 kg), the daily dose is generally 0.1-500 mg, preferably 1-100 mg, and this amount can be administered once or several times a day in divided portions.

When, for example, a "therapeutic drug of hyperlipidema" is administered to an adult (body weight 50 kg), the daily dose is generally 0.01-3000 mg, preferably 1-2000 mg, and this amount can be administered once or several times a day in divided portions.

When an HMG-CoA reductase inhibitor is administered to an adult (body weight 50 kg), the daily dose is generally 0.01-100 mg, preferably 0.5-50 mg, and this amount can be administered once or several times a day in divided portions.

When a fibrate compound is administered to an adult (body weight 50 kg), the daily dose is generally 1-2000 mg, preferably 10-1500 mg, and this amount can be administered once or several times a day in divided portions.

When a "diuretic agent" is administered to an adult (body weight 50 kg), the daily dose is generally 0.01 mg-100 g, preferably 0.05 mg-10 g, and this amount can be administered once or several times a day in divided portions.

When a xanthine derivative preparation is administered to an adult (body weight 50 kg), the daily dose is generally 0.1-100 g, preferably 0.5-10 g, and this amount can be administered once or several times a day in divided portions.

When a thiazide preparation is administered to an adult (body weight 50 kg), the daily dose is generally 0.01-2000 mg, preferably 0.05-500 mg, and this amount can be administered once or several times a day in divided portions.

When an anti-aldosterone preparation is administered to an adult (body weight 50 kg), the daily dose is generally 1-2000 mg, preferably 10-1000 mg, and this amount can be administered once or several times a day in divided portions.

When a carbonic anhydrase inhibitor is administered to an adult (body weight 50 kg), the daily dose is generally 10-5000 mg, preferably 50-2000 mg, and this amount can be administered once or several times a day in divided portions.

When a chlorobenzenesulfonamide preparation is administered to an adult (body weight 50 kg), the daily dose is generally 1-2000 mg, preferably 10-1000 mg, and this amount can be administered once or several times a day in divided portions.

The mixing ratio of compound (II) and a pharmaceutical agents for combined use can be determined appropriately depending on the administration subject, age and body weight of the administration subject, symptom, administration time, dosage form, administration method and the like.

For example, a pharmaceutical agent for combined use may be used in a proportion of 0.0001-10000 parts by weight per 1 part by weight of compound (II).

When an "insulin sensitizer other than compound (II)" is used in the angiogenesis inhibitor of the present invention, the dosage form of the "insulin sensitizer other than compound (II)" and the mixing ratio of compound (II) and the "insulin sensitizer other than compound (II)" is the same as those in the case of the aforementioned pharmaceutical agent for combined use.

### Best Mode for Carrying Out the Invention

In the following, the present invention is described in more detail by way of Reference Examples, Examples and Experimental Examples, which are not to be construed as limitative. In the following Reference Eamples, % means percent by weight unless otherwise specified. The melting point refers to that measured using a micromelting point measuring apparatus (Yanako, MP-500D) and the measures are all unamended.

The measurement data of powder X-ray diffraction were obtained using RINT1100 (Rigaku Industrial Corporation) and Cu-Kα1 ray (tube voltage:40 KV; tube current: 40 mA) as a ray source.

### Reference Example 1

To a mixed solvent of dimethyl sulfoxide (50 mL) and tetrahydrofuran (400 mL) was added sodium hydride (60% in oil, 9.04 g) at room temperature. This mixture was stirred at 50°C for 1.5 hr and allowed to cool to room temperature. To this mixture was added [2-(1,3-dioxolan-2-yl)ethyl]-triphenylphosphonium bromide (81.13 g) at room temperature. After stirring for 30 min, a solution of 4-benzyloxy benzaldehyde (30.0 g) in dimethyl sulfoxide (50 mL) was added dropwise. The reaction mixture was refluxed for 1 hr and water was added. The mixture was neutralized with 1N hydrochloric acid and extracted with ethyl acetate. The organic layer was washed successively with water and saturated brine, and dried (MgSO₄). The solvent was evaporated under reduced pressure, and the residue was subjected to silica gel column chromatography and eluted with hexane-ethyl acetate (6:1, v/v) to give (E)-2-[3-(4-benzyloxyphenyl)-2-propenyl]-1,3-dioxolane (36.03 g, yield 86%) as colorless crystals.

### Reference Example 2

A mixture of (E)-2-[3-(4-benzyloxyphenyl)-2-propenyl]-1,3-dioxolane (35.50 g), 5% palladium-carbon (40.0 g) and tetrahydrofuran (500 mL) was subjected to catalytic reduction at room temperature and 1 atm. The catalyst was filtered off and the solvent was evaporated under reduced pressure. The residue was dissolved in ethyl acetate, washed with saturated brine and dried (MgSO₄). The solvent was evaporated under reduced pressure, and the residue was subjected to silica gel column chromatography and eluted with hexane-ethyl acetate (4:1, v/v) to give 2-[3-(4-hydroxyphenyl)propyl]-1,3-dioxolane (21.9 g, yield 88%) as colorless crystals. Recrystallization from ethyl acetate-hexane gave a colorless prism crystal. melting point: 67-68°C.

### Reference Example 3

A mixture of 2-[3-(4-hydroxyphenyl)propyl]-1,3-dioxolane (21.8 g), 4-chloromethyl-5-methyl-2-phenylthiazole (26.0 g), potassium carbonate (14.5 g) and N,N-dimethylformamide (200 mL) was stirred at 90°C for 5 hr. Water was added to the reaction mixture and the mixture was extracted with ethyl acetate. The organic layer was washed with saturated brine and dried (MgSO₄), and the solvent was evaporated under reduced pressure. The obtained crystal was washed with hexane to give 4-[4-[3-(1,3-dioxolan-2-yl)propyl]phenoxymethyl]-5-methyl-2-phenylthiazole (30.98 g). The mother liquor was concentrated under reduced pressure, and the residue was subjected to silica gel column chromatography and eluted with hexane-ethyl acetate (4:1, v/v) to give 4-[4-[3-(1,3-dioxolan-2-yl)propyl]-phenoxymethyl]-5-methyl-2-phenylthiazole (4.68 g) as pale-yellow crystals (yield 35.66 g, yield 86%). Recrystallization from ethyl acetate-hexane gave a pale-yellow prism crystal. melting point: 80-81°C.

### Reference Example 4

A mixture of 4-[4-[3-(1,3-dioxolan-2-yl)propyl]phenoxymethyl]-5-methyl-2-phenylthiazole (6.0 g), 1N hydrochloric acid (60 ml) and tetrahydrofuran (60 mL) was refluxed for 5 hr. The reaction mixture was extracted with ethyl acetate. The organic layer was washed successively with water and saturated brine and dried (MgSO₄). The solvent was evaporated under reduced pressure, and the residue was subjected to silica gel column chromatography and eluted with hexane-ethyl acetate (9:1, v/v) to give 4-[4-[(5-methyl-2-phenyl-4-thiazolyl)methoxy]phenyl]butylaldehyde (4.83 g, yield 90%) as colorless crystals. Recrystallization from ethyl acetate-hexane gave a colorless prism crystal. melting point: 45-46°C.

### Reference Example 5

To a mixture of 4-[4-[(5-methyl-2-phenyl-4-thiazolyl)methoxy]phenyl]butylaldehyde (4.68 g), benzyl tributylammonium chloride (2.07 g), ethyl acetate (150 mL) and water (30 mL) was added sodium cyanide (650 mg) at room temperature. After stirring for 1.5 hr at room temperature, 2N hydrochloric acid (10 mL) was added to the reaction mixture to make the mixture acidic. The ethyl acetate layer was separated, washed successively with water (80 mL) and saturated brine (50 mL) and dried (MgSO₄). The solvent was evaporated under reduced pressure, and the residue was subjected to silica gel column chromatography and eluted with hexane-ethyl acetate (2:1, v/v) to give 2-hydroxy-5-[4-[(5-methyl-2-phenyl-4-thiazolyl)methoxy]phenyl]pentanenitrile (4.22 g, 84%). Recrystallization from ethyl acetate-hexane gave a colorless prism crystal. melting point: 101-102°C

### Reference Example 6

A mixture of (R)-(+)-α-methoxy-α-(trifluoromethyl)-phenylacetic acid (5.15 g), triethylamine (2.45 g) and anhydrous tetrahydrofuran (100 mL) was stirred at room temperature for 30 min. To the obtained mixture was added dropwise 2,4,6-trichlorobenzoyl chloride (5.37 g) at room temperature, and the mixture was stirred at room temperature for 2.5 hr. To the obtained mixture were added 2-hydroxy-5-[4-[(5-methyl-2-phenyl-4-thiazolyl)methoxy]phenyl]pentanenitrile (4.15 g) and 4-dimethylaminopyridine (1.34 g), and the mixture was stirred at room temperature for 3 hr. Water (50 mL) was added to the reaction mixture, and the mixture was extracted with ethyl acetate. The organic layer was washed successively with 1N hydrochloric acid, saturated aqueous sodium hydrogen carbonate and saturated brine and dried (MgSO₄). The solvent was evaporated under reduced pressure and the residue was subjected to silica gel column chromatography and eluted with hexane-diethyl ether (7:1, v/v) for about 12 hr. The eluate was divided into fractions of about 20 mL each using a fraction collector, and (1R)-1-cyano-4-[4-[(5-methyl-2-phenyl-4-thiazolyl)methoxy]phenyl]butyl (2R)-3,3,3-trifluoro-2-methoxy-2-phenylpropionate, which is detected as a low porality spot by thin layer chromatography, was obtained as a colorless oil (2.79 g, 43%).
¹H-NMR (CDCl₃)δ:1.62-1.74 (2H, m), 1.86-1.98 (2H, m), 2.53 (3H, s), 2.51-2.58 (2H, m), 3.57 (3H, d, J=1.0 Hz), 5.15 (2H, s), 5.50 (1H, t, J=6.6 Hz), 6.92-7.03 (4H, m), 7.37-7.49 (8H, m), 7.87-7.92 (2H, m).

### Reference Example 7

A mixture of (1R)-1-cyano-4-[4-[(5-methyl-2-phenyl-4-thiazolyl)methoxy]phenyl]butyl (2R)-3,3,3-trifluoro-2-methoxy-2-phenylpropionate (2.71 g) and 10% hydrochloric acid-methanol (100 mL; Tokyo Kasei Kogyo Co., Ltd.) was stirred in a sealed tube at room temperature for 3 days. Water was added to the reaction mixture and the mixture was extracted with ethyl acetate. The organic layer was washed with saturated brine and dried (MgSO₄). The solvent was evaporated under reduced pressure to give methyl (2R)-5-[4-[(5-methyl-2-phenyl-4-thiazolyl)methoxy]phenyl]-2-[(2R)-3,3,3-trifluoro-2-methoxy-2-phenylpropionyloxy]pentanoate as a colorless oil (2.83 g, 98%).
¹H-NMR (CDCl₃)δ: 1.50-1.63 (2H, m), 1.82-1.93 (2H, m), 2.45-2.53 (2H, m), 2.53 (3H, s), 3.65 (3H, d, J=1.0 Hz), 3.76 (3H, s), 5.15 (2H, s), 5.19 (1H, t, J=6.6 Hz), 6.90-7.00 (4H, m), 7.35-7.45 (6H, m), 7.61-7.65 (2H, m), 7.87-7.92 (2H, m).

### Reference Example 8

To a solution of methyl (2R)-5-[4-[(5-methyl-2-phenyl-4-thiazolyl)methoxy]phenyl]-2-[(2R)-3,3,3-trifluoro-2-methoxy-2-phenylpropionyloxy]pentanoate (1.80 g) in methanol (10 mL)-tetrahydrofuran (10 mL) was added dropwise 1N aqueous sodium hydroxide solution (8.7 mL) at room temperature and the mixture was stirred at 50°C for 7 hr. The reaction mixture was allowed to cool to room temperature and 1N hydrochloric acid (12 mL) and water (150 mL) were added. The mixture was extracted with ethyl acetate and the organic layer was washed with saturated brine and dried (MgSO₄). The solvent was evaporated under reduced pressure to give a colorless oil (1.74 g). A mixture of the obtained oil (1.74 g), conc. sulfuric acid (0.5 mL) and methanol (100 mL) was heated under reflux for 5 hr. The reaction mixture was concentrated under reduced pressure and ethyl acetate was added. The mixture was washed successively with saturated aqueous sodium hydrogen carbonate and saturated brine and dried (MgSO₄). The solvent was evaporated under reduced pressure, and the residue was subjected to silica gel column chromatography and eluted with hexane-ethyl acetate (3:1, v/v) to give methyl (2R)-2-hydroxy-5-[4-[(5-methyl-2-phenyl-4-thiazolyl)methoxy]phenyl]pentanoate as colorless crystals. The obtained crystals were filtrated with hexane (yield: 0.74 g, 62%, 98.1%e.e.).
¹H-NMR (CDCl₃)δ: 1.61-1.84 (4H, m), 2.53 (3H, s), 2.55-2.70 (3H, m), 3.77 (3H, s), 4.18 (1H, brs), 5.15 (2H, s), 6.93-7.01 (2H, m), 7.07-7.12 (2H, m), 7.39-7.45 (3H, m), 7.87-7.92 (2H, m).

### Reference Example 9

To a solution of methyl (2R)-2-hydroxy-5-[4-[(5-methyl-2-phenyl-4-thiazolyl)methoxy]phenyl]pentanoate (2.80 g) in methanol (40 mL) was added dropwise 1N aqueous sodium hydroxide solution (14 mL) at room temperature, and the mixture was stirred at room temperature for 1 hr. The reaction mixture was poured into water, acidified with 2N hydrochloric acid and extracted with ethyl acetate. The organic layer was washed with water and dried (MgSO₄). The solvent was evaporated under reduced pressure to give (R)-2-hydroxy-5-[4-[(5-methyl-2-phenyl-4-thiazolyl)methoxy]phenyl]pentanoic acid as colorless crystals. The obtained crystals were collected by filtration using isopropyl ether (yield: 2.24 g, 83%). melting point: 117-118°C.

### Reference Example 10

To a solution of (R)-2-hydroxy-5-[4-[(5-methyl-2-phenyl-4-thiazolyl)methoxy]phenyl]pentanoic acid (2.20 g) and triethylamine (1.23 g) in tetrahydrofuran (70 mL) was added dropwise ethyl chlorocarbonate (1.50 g) at -20°C to -15°C, and the mixture was stirred for 2 hr. 25% Aqueous ammonia (30 mL) was added and the mixture was stirred at room temperature for 1 hr. The reaction mixture was poured into water and extracted with ethyl acetate. The organic layer was washed successively with water, 2N hydrochloric acid and saturated brine, and dried (MgSO₄). The solvent was evaporated under reduced pressure to give (R)-2-ethoxycarbonyloxy-5-[4-[(5-methyl-2-phenyl-4-thiazolyl)methoxy]phenyl]pentanamide as crystals. The obtained crystals were collected by filtration using isopropyl ether (yield: 2.07 g, 80%). melting point: 130-131°C.

### Reference Example 11

(+)-5-[3-[4-[(5-Methyl-2-phenyl-4-thiazolyl)methoxy]-phenyl]propyl]-2,4-oxazolidinedione (1002 mg) was divided by a chiral column method (about 3 mg/l shot; column: CHIRALPAK AD, 10 mmφ×250 mm; mobile phase: hexane/ethanol/acetic acid=20/80/0.1; flow rate: 3.5 ml/min) and the isomers that showed long retention time were collected and concentrated to give (S)-(-)-5-[3-[4-[(5-methyl-2-phenyl-4-thiazolyl)methoxy]-phenyl]propyl]-2,4-oxazolidinedione (407 mg, 99.9%e.e.). Recrystallization from acetone-isopropyl ether gave a colorless prism crystal. melting point: 113-114°C.

### Reference Example 12

A mixture of (R)-2-ethoxycarbonyloxy-5-[4-[(5-methyl-2-phenyl-4-thiazolyl)methoxy]phenyl]pentanamide (2.00 g), 1,8-diazabicyclo[5,4,0]-7-undecene (DBU, 1.43 g) and ethanol (40 mL) was stirred at room temperature for 2 hr. The reaction mixture was poured into water and extracted with ethyl acetate. The organic layer was washed successively with water, 2N hydrochloric acid and saturated brine and dried (MgSO₄). The solvent was evaporated under reduced pressure to give (R)-(+)-5-[3-[4-[(5-methyl-2-phenyl-4-thiazolyl)methoxy]phenyl]-propyl]-2,4-oxazolidinedione as crystals (1.70 g, 94%). Recrystallization from ethyl acetate-isopropyl ether gave a colorless prism crystal (99.9%e.e.). [α]_{D}+37.3° (c=0.505, chloroform). melting point: 113-114°C.
Elemental analysis
Calculated: C,65.38; H,5.25; N,6.63
Found: C,65.30; H,5.07; N,6.68

| Data of Powder X-ray Crystal Diffraction | |
|---|---|
| Angle of Diffraction: 2θ(°) | Spacing: d value (Å) |
| 6.56 | 13.5 |
| 17.1 | 5.18 |
| 19.1 | 4.64 |
| 19.4 | 4.56 |
| 24.4 | 3.65 |

### Reference Example 13

To a solution of (R)-2-ethoxycarbonyloxy-5-[4-[(5-methyl-2-phenyl-4-thiazolyl)methoxy]phenyl]pentanamide (51.4 g) in ethanol (500 mL) was added dropwise 1,8-diazabicyclo[5,4,0]-7-undecene (DBU, 36.7 g) at 15-20°C. The mixture was stirred at 20-25°C for 2 hr and water (1 L) and 2N hydrochloric acid (150 mL) were added to the reaction mixture. The precipitated crystals were collected by filtration. The crystals were purified by silica gel column chromatography (silica gel 700 g, acetone-hexane (1:1, v/v) elution) to give (R)-(+)-5-[3-[4-[(5-methyl-2-phenyl-4-thiazolyl)methoxy]phenyl]propyl]-2,4-oxazolidinedione as crystals (44.1 g, 95%). Recrystallization from ethyl acetate-isopropyl ether gave a colorless needle crystal (>99.9%e.e.). [α]_{D}+36.7° (c=0.497, methanol). melting point: 130-131°C.
Elemental analysis
Calculated: C,65.38; H,5.25; N,6.63
Found: C,65.23; H,5.29; N,6.58

| Data of Powder X-ray Crystal Diffraction | |
|---|---|
| Angle of Diffraction: 2θ(°) | Spacing: d value (Å) |
| 8.66 | 10.2 |
| 9.44 | 9.36 |
| 12.0 | 7.34 |
| 17.4 | 5.09 |
| 18.8 | 4.71 |
| 21.8 | 4.08 |
| 29.5 | 3.03 |

### Example 1 (Production of film-coated tablets)

### [Production of coating agent]

Hydroxypropylmethyl cellulose 2910 (TC-5, 403.4 g) and polyethylene glycol 6000 (81.0 g) were dissolved in purified water (4859.1 g). Titanium oxide (54.0 g) and yellow ferric oxide (1.620 g) were dispersed in the obtained solution to give a coating agent.

### [Production of plain tablets]

The compound (7) (576.0 g), lactose (2513 g), cornstarch (356.4 g) and croscarmellose sodium (217.8 g) were charged in a fluidized-bed granulating dryer (produced by POWREX), and mixed with preheating. Then, the mixture was granulated while spraying an aqueous solution (1963 g) of hydroxypropyl cellulose (138.6 g). The obtained granules (3590 g) were milled in Power-Mill (produced by Showa Machinery Co., Ltd.) to give a pulverized powder. The obtained pulverized powder (3432 g), cornstarch (125.1 g) and magnesium stearate (17.88 g) were mixed in a tumbler mixer (produced by Showa Machinery Co., Ltd.). The obtained mixed powder (3410 g) was tabletted with a tabletting machine (produced by Kikusui Seisakusho Co., Ltd.) to give plain tablets.

### [Production of film-coated tablets]

The above coating agent was sprayed onto the obtained 27000 plain tablets in a film-coating machine (produced by POWREX) to give 27000 film-coated tablets having the following formulation and containing 16.0 mg of compound (7) per tablet.

| Formulation of tablet (composition per tablet): | |
|---|---|
| (plain tablet) | |
| 1) compound (7) | 16.0 mg |
| 2) lactose | 69.8 mg |
| 3) cornstarch | 13.75 mg |
| 4) croscarmellose sodium | 6.05 mg |
| 5) hydroxypropyl cellulose | 3.85 mg |
| 6) magnesium stearate | 0.55 mg |
| total | 110.0 mg |

| (film components) | |
|---|---|
| 7) hydroxypropylmethyl cellulose 2910 | 2.988 mg |
| 8) polyethylene glycol 6000 | 0.6 mg |
| 9) titanium oxide | 0.4 mg |
| 10) yellow ferric oxide | 0.012 mg |
| total | 114.0 mg |

### Example 2 (Production of film-coated tablets)

### [Production of plain tablets]

Film-coated tablets (27000 tablets), having the following formulation and containing 4.0 mg of compound (7) per tablet, were produced in the same manner as in Example 1 except that the amount of compound (7) and lactose used was 144.0 g and 2945 g, respectively.

| Formulation of tablet (composition per tablet): | |
|---|---|
| (plain tablet) | |
| 1) compound (7) | 4.0 mg |
| 2) lactose | 81.8 mg |
| 3) cornstarch | 13.75 mg |
| 4) croscarmellose sodium | 6.05 mg |
| 5) hydroxypropyl cellulose | 3.85 mg |
| 6) magnesium stearate | 0.55 mg |
| total | 110.0 mg |

| (film components) | |
|---|---|
| 7) hydroxypropylmethyl cellulose 2910 | 2.988 mg |
| 8) polyethylene glycol 6000 | 0.6 mg |
| 9) titanium oxide | 0.4 mg |
| 10) yellow ferric oxide | 0.012 mg |
| total | 114.0 mg |

### Example 3 (Production of film-coated tablets)

### [Production of plain tablets]

Film-coated tablets (27000 tablets), having the following formulation and containing 1.0 mg of compound (7) per tablet, were produced in the same manner as in Example 1 except that the amount of compound (7) and lactose used was 36.0 g and 3053 g, respectively.

| Formulation of tablet (composition per tablet): | |
|---|---|
| (plain tablet) | |
| 1) compound (7) | 1.0 mg |
| 2) lactose | 84.8 mg |
| 3) cornstarch | 13.75 mg |
| 4) croscarmellose sodium | 6.05 mg |
| 5) hydroxypropyl cellulose | 3.85 mg |
| 6) magnesium stearate | 0.55 mg |
| total | 110.0 mg |

| (film components) | |
|---|---|
| 7) hydroxypropylmethyl cellulose 2910 | 2.988 mg |
| 8) polyethylene glycol 6000 | 0.6 mg |
| 9) titanium oxide | 0.4 mg |
| 10 yellow ferric oxide | 0.012 mg |
| total | 114.0 mg |

### Experimental Example 1

The angiogenesis inhibitory action of compound (II) and an insulin sensitizer other than compound (II) was determined by a human microvascular endothelial cell growth inhibitory assay method.

Human microvascular endothelial cells [Human Microvascular Endothelial Cells (HMVE), (produced by KURABO INDUSTRIES LTD., Japan)] (2×10⁴/ml medium, 0.1 ml/well) in MCDB107 medium (GIBCO, US) containing 2% bovine fetal serum was dispensed to a 96 well collagen-coated microplate (produced by SUMITOMO BAKELITE CO., LTD., Japan) and a dimethylformamide (DMF) solution containing various test compounds was diluted and added to the test group. The cells were cultured at 37°C in 5% CO₂ for 3 days.

Alamar Blue was added to each well and reacted at 37°C for 4 hr. Using a fluorometer (Fluoroskan Ascent, produced by LabSystems, US), the fluorescence intensity was measured and the cell count of the well was compared with that of control. Taking the cell count of the control well as 100%, the concentration (IC₅₀) showing 50% growth inhibitory activity was calcualted. The results are shown in Table 1.

**Table 1**

| Test compound | IC₅₀ (µM) |
|---|---|
| compound (B) | 107 |
| compound (D) | 496 |
| compound (7) | 23 |

From the above-mentioned results, it is clear that the compound (II) has a superior angiogenesis inhibitory action.

### Industrial Applicability

The angiogenesis inhibitor of the present invention is useful as an agent for the prophylaxis or treatment of various diseases associated with angiogenesis, such as tumor [e.g., malignant melanoma, malignant lymphoma, cancer of digestive organ (e.g., stomach, intestine etc.), lung cancer, pancreatic cancer, esophageal cancer, breast cancer, liver cancer, ovarian cancer, uterine cancer, prostate cancer, renal cancer, bladder cancer, brain tumor, Kaposi's sarcoma, angioma, osteogenic sarcoma, myosarcoma, angiofibroma etc.], inflammatory disease (e.g., rheumarthritis, psoriasis etc.), diabetic retinopathy, atherosclerosis (including abnormal neovascularization in abnormal capillary plexus formation in outeratheromatous membrane of atheromatous arteriosclerosis) and the like.

## Claims

1. An angiogenesis inhibitor containing a compound represented by the formula 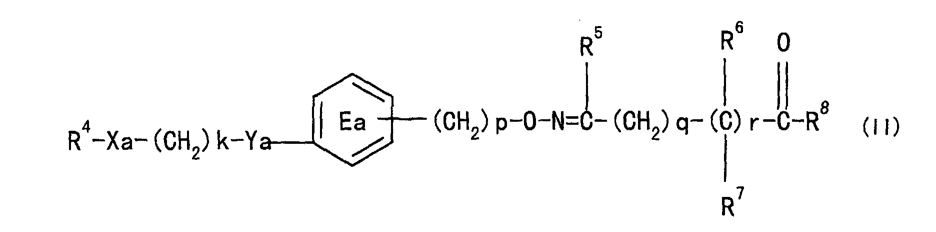 wherein
R⁴ is an optionally substituted hydrocarbon group or an optionally substituted heterocyclic group;
Xa is a bond, a group represented by the formula: -CO-, -CH(OH)- or -NR⁹- (R⁹ is a hydrogen atom or an optionally substituted alkyl group);
k is an integer of 1 to 3;
Ya is an oxygen atom, a sulfur atom, a group represented by the formula: -SO-, -SO₂- or -NR¹⁰- (R¹⁰ is a hydrogen atom or an optionally substituted alkyl group);
Ea ring is a benzene ring optionally having additional substituent(s);
p is an integer of 1 to 8;
R⁵ is a hydrogen atom, an optionally substituted hydrocarbon group or an optionally substituted heterocyclic group;
q is an integer of 0 to 6;
r is 0 or 1;
R⁸ is a hydroxy group, -OR¹¹ (R¹¹ is an optionally substituted hydrocarbon group) or -NR¹²R¹³ (R¹² and R¹³ are the same or different and each is a hydrogen atom, an optionally substituted hydrocarbon group, an optionally substituted heterocyclic group, or an acyl group, or R¹² and R¹³ are optionally bonded to form a ring); and
R⁶ and R⁷ are the same or different and each is a hydrogen atom, an optionally substituted hydrocarbon group, or R⁶ and R⁵ are optionally bonded to form a ring,
or a salt thereof.

2. The inhibitor of claim 1, which contains E-4-[4-(5-methyl-2-phenyl-4-oxazolylmethoxy)benzyloxyimino]-4-phenylbutyric acid or a salt thereof.

3. A method for inhibiting angiogenesis in a mammal, which comprises administering a compound represented by the formula 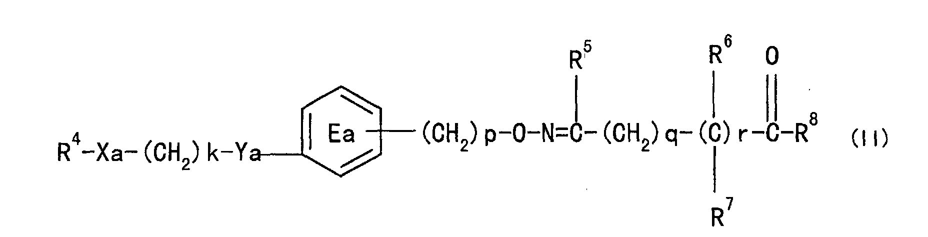 wherein
R⁴ is an optionally substituted hydrocarbon group or an optionally substituted heterocyclic group;
Xa is a bond, a group represented by the formula: -CO-, -CH(OH)- or -NR⁹- (R⁹ is a hydrogen atom or an optionally substituted alkyl group);
k is an integer of 1 to 3;
Ya is an oxygen atom, a sulfur atom, a group represented by the formula: -SO-, -SO₂- or -NR¹⁰- (R¹⁰ is a hydrogen atom or an optionally substituted alkyl group);
Ea ring is a benzene ring optionally having additional substituent(s);
p is an integer of 1 to 8;
R⁵ is a hydrogen atom, an optionally substituted hydrocarbon group or an optionally substituted heterocyclic group;
q is an integer of 0 to 6;
r is 0 or 1;
R⁸ is a hydroxy group, -OR¹¹ (R¹¹ is an optionally substituted hydrocarbon group) or -NR¹²R¹³ (R¹² and R¹³ are the same or different and each is a hydrogen atom, an optionally substituted hydrocarbon group, an optionally substituted heterocyclic group, or an acyl group, or R¹² and R¹³ are optionally bonded to form a ring); and
R⁶ and R⁷ are the same or different and each is a hydrogen atom, an optionally substituted hydrocarbon group, or R⁶ and R⁵ are optionally bonded to form a ring,
or asalt thereof, to the mammal.

4. Use of a compound represented by the formula 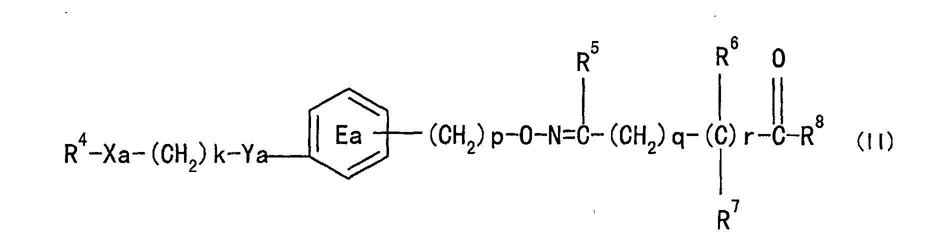 wherein
R⁴ is an optionally substituted hydrocarbon group or an optionally substituted heterocyclic group;
Xa is a bond, a group represented by the formula: -CO-, -CH(OH)- or -NR⁹- (R⁹ is a hydrogen atom or an optionally substituted alkyl group);
k is an integer of 1 to 3;
Ya is an oxygen atom, a sulfur atom, a group represented by the formula: -SO-, -SO₂- or -NR¹⁰- (R¹⁰ is a hydrogen atom or an optionally substituted alkyl group);
ring Ea is a benzene ring optionally having additional substituent(s) ;
p is an integer of 1 to 8;
R⁵ is a hydrogen atom, an optionally substituted hydrocarbon group or an optionally substituted heterocyclic group;
q is an integer of 0 to 6;
r is 0 or 1;
R⁸ is a hydroxyl group, -OR¹¹ (R¹¹ is an optionally substituted hydrocarbon group) or -NR¹²R¹³ (R¹² and R¹³ are the same or different and each is a hydrogen atom, an optionally substituted hydrocarbon group, an optionally substituted heterocyclic group, or an acyl group, or R¹² and R¹³ are optionally bonded to form a ring); and
R⁶ and R⁷ are the same or different and each is a hydrogen atom, an optionally substituted hydrocarbon group, or R⁶ and R⁵ are optionally bonded to form a ring,
or a salt thereof, for the production of an angiogenesis inhibitor.
